(19) Europäisches Patentamt
European Patent Office
Office européen des brevets

(11) **EP 4 764 220 A1**

(12) **EUROPÄISCHE PATENTANMELDUNG**

(43) Veröffentlichungstag:
**24.06.2026  Patentblatt 2026/26**

(21) Anmeldenummer: **24221942.6**

(22) Anmeldetag: **19.12.2024**

(51) Internationale Patentklassifikation (IPC):
**F04D 15/00** *(2006.01)*  **A61M 60/422** *(2021.01)*
**A61M 60/538** *(2021.01)*

(52) Gemeinsame Patentklassifikation (CPC):
**F04D 15/0088; A61M 60/178; A61M 60/237; A61M 60/422; A61M 60/538; A61M 60/546;** A61M 2205/3334

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH MA MD TN**

(71) Anmelder: **Berlin Heart GmbH**
**12247 Berlin (DE)**

(72) Erfinder:
• **PETERS, Oliver**
**12247 Berlin (DE)**
• **KIESNER, Matthias**
**15834 Rangsdorf (DE)**

(74) Vertreter: **Pfenning, Meinig & Partner mbB**
**Patent- und Rechtsanwälte**
**Joachimsthaler Straße 10-12**
**10719 Berlin (DE)**

(54) **STEUEREINHEIT UND VERFAHREN ZUR BESTIMMUNG DES DURCHFLUSSES UND DES DIFFERENZDRUCKS EINER ROTATIONSFLUIDPUMPE**

(57)   Die Erfindung bezieht sich auf ein Verfahren zur Ermittlung, des Durchflusses und/oder des Differenzdrucks einer Rotationsfluidpumpe sowie auf eine Steuereinheit (300) für eine Rotationsfluidpumpe (200), insbesondere eine Blutpumpe, wobei die Rotationsfluidpumpe (200) einen zum Fördern von Fluid um eine Rotationsachse (500) rotierbaren Rotor (240) sowie einen Stator (220) mit einer Mehrzahl von Motorspulen (221), umfasst, wobei die Steuereinheit (300) eingerichtet ist zum: Erzeugen elektrischer Antriebssignale, mit denen die Motorspulen beaufschlagt werden, Erfassen und/oder Bestimmen von Phasenstromwerten und Phasenspannungswerten für eine oder mehrere Motorspulen,
Ermittlung der Gegeninduktionsspannung aus den Phasenstromwerten und den Phasenspannungswerten, Erfassen und/oder Bestimmen der Drehzahl des Rotors Ermittlung eines Gegeninduktionskoeffizienten kv aus der Gegeninduktionsspannung und der Drehzahl, Ermitteln des Drehmomentes des Rotors aus dem Gegeninduktionskoeffizienten und den Phasenstromwerten, sowie zur
Ermittlung, des Durchflusses und/oder des Differenzdrucks der Rotationsfluidpumpe unter Berücksichtigung der Drehzahl und des Drehmomentes des Rotors.

Fig. 1

**Beschreibung**

Die Anmeldung betrifft eine Steuereinheit für eine Rotationsfluidpumpe, sowie ein Verfahren zum Bestimmen des Durchflusses und/oder des Differenzdrucks einer Rotationsfluidpumpe, insbesondere einer Blutpumpe für eine Herzunterstützungsvorrichtung.

Grundsätzlich sind Rotationsfluidpumpen, insbesondere als Blutpumpen ausgelegte Rotationsfluidpumpen, etwa für den Einsatz in einer Herzunterstützungsvorrichtung (kurz VAD für "ventricular assist device"), sowie entsprechende Steuereinheiten und Steuerverfahren bekannt. Solche Pumpen umfassen typischerweise einen Stator sowie einen zum Fördern von Fluid relativ zu dem Stator um eine Rotationsachse antreibbaren Rotor, wobei Rotor und Stator eine Elektromotoranordnung (im Folgenden auch kurz als Motor bezeichnet) bilden. Zum erwünschten Steuern oder Regeln der Rotation des Rotors ist die Kenntnis einer Winkelposition des Rotors erforderlich. In einigen Fällen kann auch die Kenntnis einer axialen und/oder radialen Position des Rotors in Bezug auf seine Rotationsachse hilfreich sein, beispielsweise für eine Positionsregelung für einen in Bezug auf den Stator in mindestens einem Freiheitsgrad aktiv magnetisch gelagerten Rotor (wobei der Rotor insbesondere berührungsfrei, also "schwebend", gelagert wird). Zur Bestimmung einer Durchflussrate und/oder eines Differenzdrucks der Pumpe wird in den meisten Fällen eine Stromaufnahme des Motors und eine Drehzahl der Pumpe ermittelt.

Bei der Konstruktion und im Betrieb solcher Pumpen sind verschiedene Anforderungen zu berücksichtigen und gegeneinander abzuwägen sowie Nachteile zu vermeiden oder zu verringern. Beispielsweise soll eine Rotationsfluidpumpe möglichst kompakt, leicht, robust sowie im Betrieb sicher, effizient und zuverlässig sein. Sie soll in vielen Fällen unter anderem die Bestimmung der Durchflussrate und/oder eines Differenzdrucks erlauben.

Aus dem Stand der Technik ist es bekannt, gesonderte Sensoren zum Erfassen der rotatorischen und/oder translatorischen Position des Rotors, wie beispielsweise Hall-sensoren vorzusehen, Hierdurch erhöhen sich in vielen Fällen die Baugröße, Masse, Komplexität und/oder Energieverbrauch der Pumpe.

Alternativ ist es bekannt, zur Positionsbestimmung und zur Drehzahlmessung die positionsabhängigen Gegeninduktionsspannungen (sogenannte "back electromotive force", BEMF), zu ermitteln, die durch Magnete des Rotors bei dessen Rotation in Motorspulen des Stators durch den Generatoreffekt erzeugt werden. Hierfür ist eine möglichst genaue Kenntnis von Modellparametern des elektrischen Modells erforderlich.

Zur Bestimmung einer Durchflussrate einer Pumpe ist es aus dem Stand der Technik bekannt, die Stromaufnahme eines Antriebsmotors der Pumpe und die Drehzahl möglichst genau zu ermitteln und aus diesen Größen unter Zuhilfenahme von Kennlinienfeldern die Durchflussrate oder den Differenzdruck zu schätzen. Den Kennlinienfeldern liegt dabei eine feste Annahme über die Motorgeometrie, insbesondere Spulengeometrie, Rotormagnetgeometrie und magnetischen Materialparametern der Rotormagnete zugrunde.

Vor dem Hintergrund des Standes der Technik liegt der Erfindung die Aufgabe zugrunde, mit einem möglichst geringen Aufwand mit einer Steuereinheit sowie einem Verfahren einen Durchfluss und/oder einen Differenzdruck einer Rotationsfluidpumpe möglichst zuverlässig und genau zu ermitteln.

Die Aufgabe wird durch die Erfindung mit den Merkmalen der unabhängigen Patentansprüche gelöst. Die abhängigen Patentansprüche stellen mögliche Weiterbildungen der Erfindung vor.

Die Erfindung bezieht sich somit auf eine Steuereinheit für eine Rotationsfluidpumpe, insbesondere eine Blutpumpe, wobei die Rotationsfluidpumpe einen zum Fördern von Fluid um eine Rotationsachse rotierbaren Rotor sowie einen Stator mit einer Mehrzahl von Motorspulen, umfasst, wobei die Steuereinheit eingerichtet ist zum:

Erzeugen elektrischer Antriebssignale, mit denen die Motorspulen beaufschlagt werden,
Erfassen und/oder Bestimmen von Phasenstromwerten und Phasenspannungswerten für eine oder mehrere Motorspulen,
Ermittlung der Gegeninduktionsspannung aus den Phasenstromwerten und den Phasenspannungswerten,
Erfassen und/oder Bestimmen der Drehzahl des Rotors
Ermittlung eines Gegeninduktionskoeffizienten kv aus der Gegeninduktionsspannung und der Drehzahl,
Ermitteln des Drehmomentes des Rotors aus dem Gegeninduktionskoeffizienten und den Phasenstromwerten, sowie
Ermittlung, insbesondere Schätzung, des Durchflusses und/oder des Differenzdrucks der Rotationsfluidpumpe unter Berücksichtigung der Drehzahl und des Drehmomentes des Rotors.

Wie weiter unten noch detailliert erläutert ist, ist die Gegeninduktionskonstante oder Back-EMF- Konstante kv, die dem Quotienten aus der Motordrehzahl und der Gegeninduktionsspannung, ($\omega / V_{motor}$) entspricht, gleich der Drehmomentkonstante $k_M$, die dem Quotienten aus dem Drehmoment und dem Motorstrom ($M / I_{motor}$) entspricht. Somit läßt sich, wenn kv aus der Motordrehzahl und der Gegeninduktionsspannung ermittelt wird, nachfolgend aus dem gemessenen Motorstrom, genauer, aus den gemessenen Phasenströmen, unter Zuhilfenahme von $k_M$ das Motordrehmoment ermitteln. Aus diesem läßt sich dann das auf das Fluid übertragene Drehmoment des Rotors ermitteln und hieraus der Durchfluß der Pumpe und/oder der Differenzdruck bestimmen.

Der Wert $k_M$ ist dabei nur solange konstant, wie die Stärke der Rotormagnete und die Rotorposition, insbesondere die Axialposition des Rotors in seinen Lagern, konstant bleibt. Die Stärke der Rotormagnete ist kurzfristig im wesentlichen nur von der Materialtemperatur der Magnete abhängig, die als weitgehend konstant angesehen werden kann, insbesondere, wenn die Pumpe eine Blutpumpe ist und der Rotor von dem geförderten Blut mit weitgehend konstanter Temperatur umspült ist. Zudem ist die Stärke der Rotormagnete von Bauteilstreuungen sowie vom Alterungszustand der Magnete abhängig. Diese nicht kurzzeitig veränderlichen Abhängigkeiten können durch Eichmessungen und Hinterlegung entsprechender Parameter in einem Schätzmodell kompensiert werden.

Der Rotor der Pumpe kann in Bezug auf den Stator in wenigstens einem Freiheitsgrad, etwa in Axialrichtung entlang der Rotationsachse, magnetisch, insbesondere aktiv magnetisch, gelagert sein. Der Rotor kann vollmagnetisch, d. h. in allen Freiheitsgraden magnetisch gelagert sein. Für eine vollmagnetische Lagerung sind beispielsweise mehrere getrennte Magnetbaugruppen (Spulen und/oder Permanentmagnete) sowohl im Stator als auch im Rotor zur Lagerung in jeweiligen Freiheitsgraden vorgesehen worden. Die Motorspulen selbst können - neben dem Erzeugen des Drehmoments für die Rotation - auch zur magnetischen Lagerung des Rotors in wenigstens einem Freiheitsgrad, insbesondere entlang der Rotationsachse, vorgesehen sein. Alternativ oder zusätzlich können gesonderte Spulen oder Spulengruppen für die magnetische Lagerung vorgesehen sein.

Die Abhängigkeit von $k_M$ von der Rotorposition wird automatisch bei der Bestimmung von kv berücksichtigt, da beide Parameter dieselbe Abhängigkeit von der Rotorposition aufweisen. Zusätzlich lässt sich jedoch aus dieser Abhängigkeit nach der Bestimmung von kv auch die Rotorposition bestimmen. Diese, insbesondere die Position des Rotors in seiner Axialrichtung, beeinflusst die hydrodynamische Effizienz des Rotors bei der Fluidförderung. Durch die zusätzliche Berücksichtigung der Rotorposition, insbesondere in Axialrichtung des Rotors, lassen sich somit der Durchfluss und/oder der Differenzdruck der Pumpe noch genauer auch unter Berücksichtigung der momentanen hydrodynamischen Effizienz bestimmen.

Um den Durchfluss und/oder den Differenzdruck besonders genau zu ermitteln, können noch Störgrößen berücksichtigt werden wie weiter unten genauer beschrieben wird.

Bei einer möglichen Implementierung der Erfindung kann vorgesehen sein, dass die Steuereinheit eingerichtet ist, im Betrieb wiederholt, insbesondere regelmäßig, den Gegeninduktionskoeffizienten kv aus der Gegeninduktionsspannung und der Drehzahl zu ermitteln , und damit insbesondere Veränderungen der Motoreffizienz und/oder der Rotorposition und/oder der Rotortemperatur und/oder der Alterung der Rotormagnete und/oder produktionsbedingte Bauteilstreuungen mit Auswirkung auf die Motoreffizienz bei der Rotordrehmomentbestimmung zu berücksichtigen. Weitere Einflüsse auf die Systemeffizienz, die die Motoreffizienz nicht direkt beeinflussen, wie beispielsweise veränderliche Widerstände der Anschlussleistungen oder von Steckkontakten, werden bei der Drehmomentbestimmung über kv ebenfalls berücksichtigt.

Der Gegeninduktionskoeffizienten kv kann beispielsweise etwa wenigstens 6 mal pro Minute oder wenigstens 3 mal pro Minute ermittelt werden.

Dadurch, dass der Gegeninduktionskoeffizient kv im Betrieb wiederholt ermittelt wird, können die wesentlichen, kurzzeitig veränderlichen Parameter, die einen Einfluss auf die Bestimmung des Pumpendurchflusses und/oder des Differenzdrucks haben, ständig aktuell berücksichtigt werden. Hierdurch wird auch eine genaue Regelung von Durchfluss und/oder Differenzdruck der Pumpe erleichtert. Es kann auch eine zeitlich gemittelte Rotorposition zuverlässig ermittelt werden, die auf diesem Weg langzeitstabiler und auch deutlich empfindlicher zu messen ist als bei einer Ermittlung der Rotorposition durch Wirbelstromsensoren oder Magnetfeldsensoren, die in vielen Fällen für eine Schweberegelung des Rotors genutzt werden. Die hydrodynamische Effizienz des Rotors kann somit, wie oben ausgeführt, laufend in einfacher und driftarmer Weise ermittelt und mitberücksichtigt werden.

Es kann weiter vorgesehen sein, dass die Steuereinheit eingerichtet ist, aus dem Gegeninduktionskoeffizienten und den Phasenströmen ein Motorspulenangangsmoment zu bestimmen, und aus diesem durch Berücksichtigung von nicht hydrodynamischen Bremsmomenten, insbesondere Wirbelstromverlusten und/ oder Hystereseverlusten, das tatsächlich auf das zu fördernde Fluid wirkende Drehmoment zu bestimmen.

Das aus dem Gegeninduktionskoeffizienten und den Phasenströmen ermittelte Drehmoment ist ein insgesamt zwischen Statorspulen und Rotor wirkendes Drehmoment. Dieses entspricht zwar etwa dem auf das Fluid wirkenden Moment, jedoch sind dabei parasitäre Kräfte oder Momente, wie Bremsmomente durch Wirbelstromwirkungen, beispielsweise in der Gehäusewand oder in Wirbelstromsensoren der Pumpe, und/ oder Hystereseverluste nicht berücksichtigt. Diese parasitären Drehmomente können in Abhängigkeit von den übrigen Größen, insbesondere von den Phasenströmen und/oder der Axialposition des Rotors und/oder der Drehzahl bestimmt und bei der Ermittlung des Durchflusses und/oder des Differenzdrucks berücksichtigt werden. Die Wirbelstromverluste sind dabei nicht nur auf Wirbelströme im Stator, beispielsweise in den Wicklungen oder im Rückflusseisen, begrenzt. Auch Wirbelströme im Pumpengehäuse und insbesondere in der Fluidtrennwand zwischen Rotor und Stator können berücksichtigt und abgezogen werden.

Es kann zudem vorgesehen sein, dass

die Steuereinheit eingerichtet ist zum:

Erzeugen eines Steuersignals für mindestens eine der Mehrzahl von Motorspulen, wobei das Steuersignal mit einem Modulationssignal beaufschlagt wird,

Erfassen mindestens eines Messsignals, entsprechend einem durch die mindestens eine der Mehrzahl von Motorspulen fließenden Strom und/oder einer an der mindestens einen der Mehrzahl von Motorspulen anliegenden Spannung, Demodulieren des mindestens einen Messsignals zum Bestimmen mindestens eines veränderlichen charakteristischen elektrischen Widerstands einer elektrischen Anordnung, umfassend die mindestens eine der Mehrzahl von Motorspulen und eine zum Verbinden der mindestens einen der Mehrzahl von Motorspulen mit der Steuereinheit eingerichtete Zuleitung.

Die Zuleitung bzw. jede der zum Verbinden einer jeweiligen Motorspule mit der Steuereinheit eingerichtete Zuleitung umfasst typischerweise wenigstens eine leitende Ader (optional mehrere leitende Adern) und vorzugsweise wenigstens einen Kontakt, etwa einen Steckkontakt, zum Verbinden der Ader mit der Pumpe und/oder der Steuereinheit. Die Kontakte können fest und/oder lösbar ausgeführt sein.

Es wurde festgestellt, dass die Kenntnis des veränderlichen charakteristischen elektrischen Widerstands der genannten Art, im Folgenden mitunter auch kurz als Phasenwiderstand bezeichnet, nützlich ist für ein genaues Bestimmen der Induktionsspannungen, insbesondere mit hinreichender Genauigkeit für eine genaue Bestimmung des Drehmomentes des Rotors oder für eine zuverlässige Regelung der Rotation und/oder Position des Rotors auf Grundlage der Positionsabhängigkeit der Induktionsspannungen. Das Messprinzip mit Beaufschlagen des Steuersignals mit dem Modulationssignal und Demodulieren des mindestens einen Messsignals ermöglicht eine genaue Bestimmung des Phasenwiderstandes, wobei verschiedene Störeinflüsse - einschließlich Variation der Induktivität, Gegeninduktion und/oder Wirbelstromverluste im Betrieb des Motors -bei dieser Messung unterdrückt werden. Die vorgeschlagene Steuereinheit ermöglicht es daher gemäß den obenstehenden Überlegungen, eine besonders kompakte, leichte und robuste Rotationsfluidpumpe vorzusehen und diese besonders sicher, effizient und zuverlässig zu betreiben. Das Beaufschlagen des Steuersignals mit dem Modulationssignal und Demodulieren des mindestens einen Messsignals eignet sich insbesondere für ein genaues Bestimmen des Phasenwiderstands bzw. von Änderungen des Phasenwiderstands über Zeitskalen, die erheblich länger sind als eine Rotationsperiode des Rotors und/oder ein Inverses einer Drehfelddrehzahl (wie weiter unten definiert) im Betrieb, beispielsweise über Zeitintervalle, die größer sind als eine Sekunde, zum Beispiel ca. 1 bis 10 s bei einer Drehfelddrehzahl von beispielsweise 0,1 bis 1 kHz.

Die Steuereinheit kann dazu eingerichtet sein, das Steuersignal unter Verwendung von Pulsbreitenmodulation zu erzeugen. Die Steuereinheit kann dann weiter dazu eingerichtet sein, das Steuersignal mit dem Modulationssignal durch Variation der Pulsbreitenmodulation, insbesondere durch Aufaddieren einer Signalform, insbesondere eines Rechtecksignals, zu beaufschlagen. Die Steuereinheit kann ebenso dazu eingerichtet sein, einen durch das Beaufschlagen des Steuersignals mit dem Modulationssignal verursachten Modulationsanteil des Messsignals mittels Demodulation, insbesondere Synchrondemodulation, zu erfassen.

Es kann weiter vorgesehen sein, dass die Steuereinheit eingerichtet ist zum Erfassen mindestens eines Messsignals, entsprechend einem durch mindestens eine der Mehrzahl von Motorspulen fließenden Strom und/oder einer an der mindestens einen der Mehrzahl von Motorspulen anliegenden Spannung, Bestimmen, basierend auf dem mindestens einen Messsignal und einem thermischen Modell, einer Änderung einer Temperatur wenigstens eines Teils der Rotationsfluidpumpe, umfassend die mindestens eine der Mehrzahl von Motorspulen, über ein Messintervall; und Bestimmen, basierend auf der bestimmten Änderung der Temperatur, eines Wertes oder einer Änderung mindestens eines veränderlichen charakteristischen elektrischen Widerstands einer elektrischen Anordnung, umfassend die mindestens eine der Mehrzahl von Motorspulen und eine zum Verbinden der mindestens einen der Mehrzahl von Motorspulen mit der Steuereinheit eingerichtete Zuleitung.

Auch mittels dieser weiteren Methode zum Bestimmen des Phasenwiderstands wird es ermöglicht, eine besonders kompakte, leichte und robuste Rotationsfluidpumpe vorzusehen und für diese möglichst genau einen Durchfluss und/oder einen Differenzdruck zu bestimmen. Das Bestimmen der Temperaturänderung basierend auf dem mindestens einen Messsignal und dem thermischen Modell eignet sich insbesondere für ein genaues Bestimmen des Phasenwiderstands bzw. von Änderungen des Phasenwiderstands über kürzere Zeitskalen als die oben für die demodulationsbasierte Methode genannten Zeitskalen; solche Änderungen können etwa durch plötzlichen Impulseintrag, etwa durch externe Schläge oder Beschleunigungen der Pumpe, verursacht werden. Durch einen solchen Impulseintrag kann - bedingt durch die erforderlichen Regelvorgänge - die Temperatur der Motorspulen kurzfristig signifikant ansteigen und sich damit der Phasenwiderstand entsprechend ändern. Auch bei einem Startvorgang des Motors, der bei einem magnetisch gelagerten und entsprechend vorgespannten Rotor mit einem Ablösen des Rotors von einer Gehäusewand einhergeht, kann aufgrund der dafür erforderlichen hohen Leistung eine schnelle Temperaturerhöhung erfolgen.

Die Änderung des Phasenwiderstands aufgrund der kurzfristigen Temperaturänderung kann bei der Bestimmung der Gegeninduktionskonstanten und des Drehmoments des Rotors der Pumpe und somit bei der Schätzung des Durchflusses oder eines Differenzdrucks der Pumpe kurzfristig berücksichtigt werden.

Außerdem kann die Steuereinheit dazu eingerichtet sein, den mindestens einen veränderlichen charakteristischen elektrischen Widerstand in einem Normalbetrieb der Rotationsfluidpumpe wiederholt für jeweils ein erstes Messintervall durch Demodulieren des mindestens einen Messsignals zu bestimmen, und
eine Änderung des mindestens einen veränderlichen charakteristischen elektrischen Widerstands über ein zweites Messintervall, das kürzer ist als das erste Messintervall, basierend auf der bestimmten Änderung der Temperatur zu bestimmen, insbesondere in Antwort auf ein Erfassen eines gegenüber dem Normalbetrieb erhöhten Leistungseintrags in die Rotationsfluidpumpe.

Die Steuereinheit kann dazu eingerichtet sein, den mindestens einen veränderlichen charakteristischen elektrischen Widerstand wiederholt für jeweils ein erstes Messintervall durch Demodulieren des mindestens einen Messsignals zu bestimmen, insbesondere in einem Normalbetrieb der Rotationsfluidpumpe. Der Normalbetrieb stellt dabei einen normalen Betriebszustand ohne momentan erhöhten Leistungseintrag dar. Die Steuereinheit kann dazu eingerichtet sein, eine Änderung des mindestens einen veränderlichen charakteristischen elektrischen Widerstands über ein zweites Messintervall basierend auf der bestimmten Änderung der Temperatur zu bestimmen, beispielsweise in Antwort auf ein Erfassen eines gegenüber dem Normalbetrieb erhöhten Leistungseintrags in die Rotationsfluidpumpe (im Folgenden auch als Ausnahmebetrieb bezeichnet). Ein wiederholtes Bestimmen basierend auf der Temperaturänderung über das zweite Messintervall kann auch im Normalbetrieb erfolgen. Das zweite Messintervall ist vorzugsweise kürzer als das erste Messintervall. Besonders vorteilhaft können die beschriebenen Messungen über das erste Messintervall durch Demodulieren und über das zweite Messintervall basierend auf der Temperaturänderung miteinander kombiniert werden. Somit ist eine Bestimmung des Phasenwiderstands bzw. seiner Änderungen mit guter Genauigkeit über sowohl kürzere als auch längere Zeitskalen möglich, wodurch auf langsamere und schnellere Änderungen entsprechend reagiert werden kann.

Das Messsignal umfasst vorzugsweise - als vektorielles Messsignal - Komponenten entsprechend den durch jede der Mehrzahl von Motorspulen fließenden Ströme und den an jeder der Mehrzahl von Motorspulen anliegenden Spannungen. Die Steuereinheit kann zum sequentiellen und/oder gleichzeitigen Erfassen der genannten Komponenten eingerichtet sein.

Es soll darauf hingewiesen werden, dass das als Modulationssignal bezeichnete Signal ein in Amplitude und/oder Frequenz und/oder Phase konstantes Signal sein kann, das also für sich genommen keine aufmodulierte Information enthält. Eine Amplituden- und Phasenmodulation des mit dem Modulationssignal beaufschlagten Steuersignals ergibt sich dann durch den Motor selbst, der also als Modulator wirkt. Das erfassbare Messsignal ist somit ein moduliertes Signal, dem durch Demodulieren Information insbesondere über den Phasenwiderstand in der beschriebenen Weise entnehmbar ist.

Es kann vorgesehen sein, dass der Stator eine Mehrzahl von Motorphasen, umfassend jeweils mindestens eine der Mehrzahl von Motorspulen, umfasst und die Steuereinheit dazu eingerichtet ist,

nacheinander eine Mehrzahl von Modulationszuständen vorzugeben, wobei in jedem Modulationszustand ein jeweiliger Satz von Motorphasen gegenphasig oder phasenverschoben mit dem Modulationssignal beaufschlagt wird und
wobei das Messsignal im jeweiligen Modulationszustand an dem jeweils mit dem Modulationssignal beaufschlagten Satz von Motorphasen abgegriffen wird.
Auf diese Weise können alle relevanten Komponenten des Messsignals zum Bestimmen aller relevanten Phasenwiderstände des Motors erfasst werden.

Eine Modulationsfrequenz des Modulationssignals kann weniger als eine Drehfelddrehzahl eines die Rotation des Rotors verursachenden Drehfelds, vorzugsweise weniger als 50 Hz, betragen. Die Drehfelddrehzahl ist dabei insbesondere als Produkt aus einer Rotationsfrequenz des Rotors und einer Anzahl von Polpaaren des Rotors darstellbar. Durch eine solche Wahl der Modulationsfrequenz kann - insbesondere in Verbindung mit hinreichend geringer Amplitude des Modulationssignals - ein Störeinfluss des Modulationssignals auf das Steuersignal vermieden bzw. minimiert werden. Die Drehfelddrehzahl kann beispielweise im Bereich von 0,1 bis 1 kHz liegen.

Zumindest ein Anteil des erfassten Messsignals und/oder eine basierend auf dem Messsignal bestimmte Größe (insbesondere ein Spannungs- und/oder Stromwert) kann zum Bestimmen des mindestens einen veränderlichen charakteristischen elektrischen Widerstands zeitlich gemittelt und/oder kumuliert werden, insbesondere über ein Intervall von mindestens 1 s und/oder höchstens 10 s. Auf diese Weise kann ein erhöhter Signal-Rauschabstand und eine entsprechend genaue Messung des Phasenwiderstandes erzielt werden. Das Intervall kann zur Laufzeit an die momentanen Anforderungen angepasst werden; beispielsweise kann eine schnelle, kurzzeitige Messung mit reduziertem Intervall bei erhöhter Modulationsamplitude durchgeführt werden.

Es kann beispielsweise auch vorgesehen sein, dass die Steuereinheit dazu eingerichtet ist, aus dem Gegeninduktionskoeffizienten eine Lagerposition des Rotors in seiner Axialrichtung, auch Axialposition des Rotors genannt, zu ermitteln und diese insbesondere bei der Ermittlung oder Schätzung des Flusses und/oder des Differenzialdrucks der Rotationsfluidpumpe und/oder bei der Lagerregelung zu berücksichtigen.

Die Axialposition des Rotors beeinflusst, wie bereits weiter oben ausgeführt wurde, die hydrodynamische Effizienz des Rotors bei der Fluidförderung. Auch eine Abweichung der Rotorposition in anderen als axialen Richtungen, beispielsweise in radialer Richtung, kann die hydrodynamische Effizienz des Rotors beeinflussen und aus diesem Grund ermittelt und berücksichtigt werden. Durch die Berücksichtigung der Rotorposition, insbesondere in Axialrichtung des Rotors, in manchen Fällen zusätzlich auch in radialer Richtung oder auch der Rotorverkippung, lassen sich somit der Durchfluss und/oder der Differenzdruck der Pumpe noch genauer bestimmen.

Eine weitere Ausführungsform kann vorsehen, dass die Steuereinheit eine Zuordnungseinheit aufweist, die dazu eingerichtet ist, aufgrund eines Zuordnungsmodells einem ermittelten Drehmoment und einer Drehzahl des Rotors, insbesondere unter Berücksichtigung einer Lagerposition des Rotors und/oder eines Viskositätswertes des geförderten Fluides einen Durchfluss und/oder einen Differenzdruck der Rotationsfluidpumpe zuzuordnen, wobei das Zuordnungsmodell insbesondere eine Zuordnungstabelle, eine Zuordnungsfunktion, einen Beobachter oder ein selbstlernendes System, insbesondere ein neuronales Netz, umfasst.

Zur Eichung und/oder zum Training eines Zuordnungsmodells können Versuchsmessungen unter variierten Bedingungen durchgeführt werden, bei denen Referenzmesswerte ermittelt werden. An diesen Werten oder Wertekombinationen kann das Zuordnungsmodell trainiert werden. Im Betrieb kann die Steuereinheit dem Zuordnungsmodell ermittelte Werte liefern, worauf in Echtzeit ein Arbeitspunkt ermittelt wird, der am besten zu den Messwerten passt und diesen Messwerten einen Differenzdruck und/oder einen Fluiddurchfluss zuordnet. Bei dieser Zuordnung kann auch eine zeitliche Entwicklung in Form der jeweils zuletzt ermittelten Arbeitspunkte mitberücksichtigt werden.

Es kann ein Modell an Messdaten angefittet werden, um gegebenenfalls auch Daten zu interpolieren. Das Modell kann auch komplexe Zusammenhänge abbilden, wenn beispielsweise Kalman-Filter, Beobachter oder neuronale Netze genutzt werden.

Die Steuereinheit kann dazu eingerichtet sein, die Rotation des Rotors und/oder eine rotatorische und/oder translatorische Position des Rotors basierend auf dem Messsignal und einem Modell wenigstens eines Teils der Rotationsfluidpumpe wenigstens zu erfassen, vorzugsweise auch zu regeln, wobei das Modell insbesondere den mindestens einen veränderlichen charakteristischen elektrischen Widerstand umfasst. Als Regeln wird hier ein Regeln mit Rückkopplung (also "closed-loop control") bezeichnet. Die Steuereinheit kann zur entsprechenden Ansteuerung der Motorspulen, etwa mittels Blockkommutierung, Sinuskommutierung, Raumzeigermodulation und/oder Vektorregelung (field-oriented control, FOC), eingerichtet sein. Beispielsweise kann bei Ansteuerung mittels einer Vektorregelung unter Verwendung eines durch Park-Transformation erhaltenen dq-Systems oder bei Sinuskommutierung mittels inverser Park-Transformation die Rotation des Rotors über den Drehmomentstrom ($I_q$), eine Axialkraft zur Positionsregelung entlang der Rotationsachse über die Feldkomponente ($I_d$) geregelt werden.

Die Steuereinheit kann auch dazu eingerichtet sein, basierend auf dem mindestens einen veränderlichen charakteristischen elektrischen Widerstand einen Verbindungszustand zwischen der Rotationsfluidpumpe und der Steuereinheit und/oder einen Defektzustand der Rotationsfluidpumpe und/oder der Zuleitung zu erfassen. Durch Herstellen bzw. Lösen der Verbindung (also Ändern des Verbindungszustands) oder Defekte (wie beispielsweise Aderbruch in der Pumpe und/oder der Zuleitung) kommt es zu Änderungen des Phasenwiderstands, die mit den vorgeschlagenen Methoden erfasst werden können. Das Erfassen des Verbindungszustands kann beispielsweise einen Pull-Up/Down-Widerstand in der Steckverbindung ersetzen und somit den Hardwareaufwand verringern. Zudem werden in diesem Fall dieselben Leitungen und Sensoren verwendet wie für den Betrieb der Pumpe, so dass dieses Erfassen gegenüber einem Ansatz mit separaten Pins die Zuverlässigkeit verbessern kann. Das Erfassen von Defektzuständen kann die Sicherheit der Pumpe verbessern (etwa kann in Antwort auf ein solches Erfassen eine Warnung ausgegeben und/oder auf einen alternativen Betriebsmodus umgeschaltet werden).

Weiter bezieht sich die Erfindung auf ein Pumpensystem, umfassend
eine Rotationsfluidpumpe, umfassend einen zum Fördern von Fluid um eine Rotationsachse rotierbaren Rotor sowie einen Stator mit einer Mehrzahl von Motorspulen, und eine Steuereinheit der oben beschriebenen Art.

In dem vorgeschlagenen Pumpensystem entfaltet die Steuereinheit ihre oben genannten sowie weitere Wirkungen und Vorteile und erlaubt durch das Trainieren oder Einrichten des Zuordnungsmodells auf den konkreten Rotor und den Stator der Pumpe eine genaue Bestimmung des Durchflusses und/oder des Differentialdrucks der Rotationsfluidpumpe. Das Pumpensystem kann insbesondere als Blutpumpensystem mit VAD mit großen Vorteilen eingesetzt werden.

Die Erfindung bezieht sich weiterhin auf ein Verfahren zum Ermitteln eines Durchflusses und/oder eines Differenzdrucks einer Rotationsfluidpumpe, insbesondere einer Blutpumpe, wobei die Rotationsfluidpumpe (200) einen zum Fördern von Fluid um eine Rotationsachse rotierbaren Rotor sowie einen Stator mit einer Mehrzahl von Motorspulen, umfasst, mittels einer Steuereinheit, wobei die Steuereinheit

elektrische Antriebssignale erzeugt, mit denen die Motorspulen beaufschlagt werden,

Phasenstromwerte und Phasenspannungswerte für eine oder mehrere Motorspulen erfasst oder bestimmt, eine Gegeninduktionsspannung aus den Phasenstromwerten und den Phasenspannungswerten ermittelt, die Drehzahl des Rotors erfasst oder bestimmt, einen Gegeninduktionskoeffizienten kv aus der Gegeninduktionsspannung und der Drehzahl ermittelt, das Drehmoment des Rotors aus dem Gegeninduktionskoeffizienten und den Phasenstromwerten ermittelt, sowie den Durchfluss und/oder den Differenzdruck der Rotationsfluidpumpe unter Berücksichtigung der Drehzahl und des Drehmomentes des Rotors ermittelt, insbesondere schätzt.

Die Vorteile und Einsatzmöglichkeiten eines solchen Verfahrens ergeben sich aus den Vorteilen, die weiter oben im Zusammenhang mit einer für dieses Verfahren eingerichteten Steuereinheit bereits beschrieben worden sind.

Das Verfahren kann beispielsweise dadurch weitergebildet werden, dass die Steuereinheit im Betrieb wiederholt, insbesondere regelmäßig, den Gegeninduktionskoeffizienten kv aus der Gegeninduktionsspannung und der Drehzahl ermittelt, und damit insbesondere Veränderungen der Moteffizienz und/oder der Rotorposition und/oder der Rotortemperatur und/oder der Alterung der Rotormagnete bei der Rotordrehmomentbestimmung berücksichtigt. Auch die Vorteile dieser Ausprägung des Verfahrens sind bereits weiter oben geschildert worden.

Eine weitere mögliche Realisierung kann vorsehen, dass die Steuereinheit

ein Steuersignal für mindestens eine der Mehrzahl von Motorspulen erzeugt, wobei das Steuersignal mit einem Modulationssignal beaufschlagt wird, mindestens ein Messsignal erfasst, das einen durch die mindestens eine der Mehrzahl von Motorspulen fließenden Strom und/oder eine an der mindestens einen der Mehrzahl von Motorspulen anliegende Spannung repräsentiert,

mindestens ein Messsignal zum Bestimmen mindestens eines veränderlichen charakteristischen elektrischen Widerstands einer elektrischen Anordnung demoduliert, wobei die Anordnung mindestens eine der Mehrzahl von Motorspulen und eine zum Verbinden der mindestens einen der Mehrzahl von Motorspulen mit der Steuereinheit eingerichtete Zuleitung umfasst, und/oder, dass die Steuereinheit:

mindestens ein Messsignal erfasst, das einen durch mindestens eine der Mehrzahl von Motorspulen fließenden Strom und/oder eine an der mindestens einen der Mehrzahl von Motorspulen anliegende Spannung repräsentiert,

basierend auf dem mindestens einen Messsignal und einem thermischen Modell eine Änderung einer Temperatur wenigstens eines Teils der Rotationsfluidpumpe, über ein Messintervall bestimmt, wobei der mindestens eine Teil wenigstens eine der Mehrzahl von Motorspulen umfasst; und

basierend auf der bestimmten Änderung der Temperatur, einen Wert oder eine Änderung mindestens eines veränderlichen charakteristischen elektrischen Widerstands einer elektrischen Anordnung bestimmt, wobei die elektrische Anordnung die mindestens eine der Mehrzahl von Motorspulen und eine zum Verbinden der mindestens einen der Mehrzahl von Motorspulen mit der Steuereinheit eingerichtete Zuleitung umfasst.

Bei dem Verfahren kann außerdem vorgesehen sein, dass die Steuereinheit

den mindestens einen veränderlichen charakteristischen elektrischen Widerstand in einem Normalbetrieb der Rotationsfluidpumpe wiederholt für jeweils ein erstes Messintervall durch Demodulieren des mindestens einen Messsignals bestimmt, und

eine Änderung des mindestens einen veränderlichen charakteristischen elektrischen Widerstands über ein zweites Messintervall, das kürzer ist als das erste Messintervall, basierend auf der bestimmten Änderung der Temperatur bestimmt, insbesondere nach dem Erfassen eines gegenüber dem Normalbetrieb erhöhten Leistungseintrags in die Rotationsfluidpumpe.

Weiter kann das Verfahren dadurch realisiert werden, dass der Stator eine Mehrzahl von Motorphasen, umfassend jeweils mindestens eine der Mehrzahl von Motorspulen, umfasst und die Steuereinheit nacheinander eine Mehrzahl von Modulationszuständen vorgibt, wobei in jedem Modulationszustand ein jeweiliger Satz von Motorphasen gegenphasig oder phasenverschoben mit dem Modulationssignal beaufschlagt wird und wobei das Messsignal im jeweiligen Modulationszustand an dem jeweils mit dem Modulationssignal beaufschlagten Satz von Motorphasen abgegriffen wird.

Das Verfahren kann weiter dadurch implementiert werden, dass die Steuereinheit aus dem Gegeninduktionskoeffizienten eine Lagerposition des Rotors in seiner Axialrichtung ermittelt und diese insbesondere bei der Ermittlung oder Schätzung des Flusses und/oder des Differenzialdrucks der Rotationsfluidpumpe und/oder bei der Lageregelung berücksichtigt.

Die zuletzt beschriebenen Ausführungsformen des Verfahrens sind gemeinsam mit den Vorteilen solcher Verfahren bereits weiter oben im Zusammenhand mit der für solche Verfahren eingerichteten Steuereinheit diskutiert worden.

Im Folgenden wird die Erfindung anhand von Ausführungsbeispielen in Figuren einer Zeichnung gezeigt und anschließend beschrieben. Dabei zeigen, jeweils schematisch,

FIG. 1: ein Pumpensystem einschließlich einer Rotationsfluidpumpe in Längsschnittansicht,

FIG. 2: eine Schaltskizze eines Teils der Rotationsfluidpumpe nach FIG. 1,

FIG. 3: eine Prinzipskizze eines Verfahrens nach einem Beispiel,

FIG. 4: einen Teil des Pumpensystems nach FIG. 1,

FIG. 5: eine Prinzipskizze eines Verfahrens nach einem weiteren Beispiel,

FIG. 6: ein Verfahren zur Bestimmung der Durchflussrate und/oder eines Differenzdrucks,

FIG. 7: eine besondere Ausprägung des Verfahrens, das in der Figur 6 dargestellt ist, mit der Berücksichtigung weiterer Einflussgrößen,

FIG. 8: eine erste schematische Darstellung einer Vorrichtung nach dem marktüblichen Stand der Technik zur Bestimmung [A1] einer Durchflussrate,

FIG. 9: eine zweite, etwas detailliertere Darstellung eines Verfahrens nach dem Stand der Technik zur Bestimmung eines Differenzdrucks sowie einer Durchflussrate, sowie

FIG. 10: eine schematische Darstellung eines Verfahrensablaufs gemäß der Erfindung zur genaueren Bestimmung eines Differenzdrucks sowie einer Durchflussrate, wobei der Verfahrensablauf in der erfindungsgemäßen Steuereinheit seine Entsprechung findet.

Gleiche Bezugszeichen in den Zeichnungen bezeichnen jeweils gleiche oder zumindest funktional gleiche Teile. Die Bezugszeichen können teilweise ausgelassen werden, wenn die entsprechenden Merkmale bereits in einer anderen Zeichnung gezeigt und im Zusammenhang mit dieser beschrieben werden.

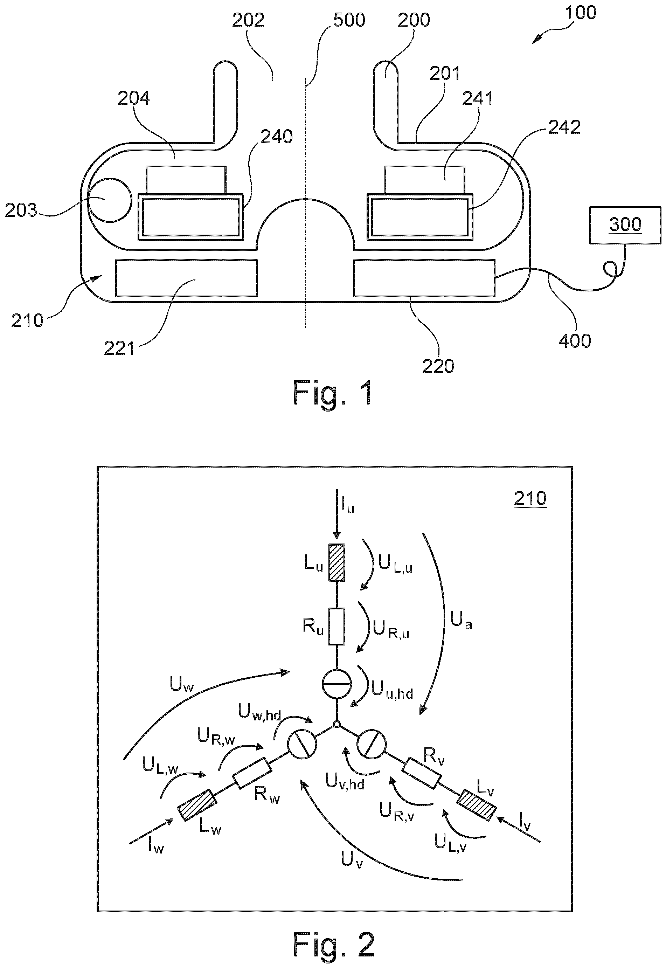

In der FIG. 1 ist ein Pumpensystem 100 dargestellt. Dieses umfasst eine Steuereinheit 300 und eine Rotationsfluidpumpe 200. Die Rotationsfluidpumpe 200 weist einen zum Fördern von Fluid um eine Rotationsachse 500 drehbaren Rotor 240 sowie einen Stator 220 mit einer Mehrzahl von Motorspulen 221 auf. Stator 220 und Rotor 240 sind Bestandteile eines Motors 210, im gezeigten Beispiel eines Axial/radialflussmotors. Der Anmeldungsgegenstand ist jedoch nicht auf derartige Motoren beschränkt und umfasst ferner beispielsweise Radialflussmotoren. Der Rotor 240 weist eine Rotormagnetanordnung 242 mit einem oder mehreren Permanentmagneten auf, die zum Wechselwirken mit den Motorspulen 221 des Stators 220 zum Erzeugen eines Drehmoments bezüglich der Rotationsache 500 eingerichtet ist.

Die Rotationsfluidpumpe 200 ist als implantierbare Blutpumpe, speziell als VAD bzw. teil eines VAD-Systems, ausgelegt, wobei das geförderte Fluid Blut ist. Auch wenigstens teilweise extrakorporale Ausführungen sind denkbar. Die Steuereinheit 300 ist eine externe (insbesondere extrakorporale), mit der Blutpumpe 200 mittels einer Driveline 400 verbindbare Steuereinheit 300. Die Steuereinheit 300 kann jedoch auch ganz oder teilweise in die Blutpumpe 200 integriert und/oder mit dieser implantierbar sein. Es kann auch vorgesehen sein, dass die Steuereinheit 300 sowohl an der Blutpumpe 200 angeordnete als auch extern/extrakorporal angeordnete Teile umfasst. Die Rotationsfluidpumpe 200 ist nicht auf eine Blutpumpe beschränkt und kann alternativ beispielsweise zum Fördern von Wasser, Öl oder einem anderen Fluid vorgesehen sein. Die beispielhaft gezeigte Pumpe 200 ist eine Zentrifugalpumpe, jedoch ist der Anmeldungsgegenstand nicht darauf beschränkt und umfasst etwa auch Radialpumpen oder Mischformen aus den genannten Pumpentypen.

Der Stator 220 ist an einem Gehäuse 201 der Pumpe angeordnet. Das Gehäuse 201 umfasst einen Fluideinlass 202 und einen Fluidauslass 203, die mit einem Blutgefäß und/oder einem Herzen fluidisch verbindbar sind. Zum Fördern des Fluids vom Fluideinlass 202 zum Fluidauslass 203 umfasst der Rotor 240 eine Beschaufelung 241, beispielsweise mit einzelnen Förderelementen in der Form von Schaufeln.

Der Rotor 240 ist innerhalb einer Kavität 204 des Gehäuses 201 berührungsfrei magnetisch gelagert. Hierzu umfassen Stator 220 und Rotor 240 geeignete Magnetanordnungen oder Magnetlager. Beispielsweise kann eine ringförmige Rotormagnetanordnung 242 im Rotor 240 zum Lagern des Rotors 240 entlang einer durch die Rotationsachse definierten Axialrichtung mit den Motorspulen 221 des Stators 220 wechselwirken, wobei die Motorspulen 221 zur Regelung der rotatorischen und/oder translatorischen Position des Rotors 240 ansteuerbar sind (etwa mittels Vektorregelung wie oben beschrieben).

Die Steuereinheit 300 ist eingerichtet zum Erfassen eines mehrkomponentigen (vektoriellen) Messsignals, umfassend Komponenten, die den durch jede der Mehrzahl von Motorspulen 221 fließenden Strömen und den an jeder der Mehrzahl von Motorspulen 221 anliegenden Spannungen entsprechen. Die Steuereinheit 200 kann zum sequentiellen und/oder gleichzeitigen Erfassen der genannten Komponenten eingerichtet sein.

Basierend auf den Messsignalen sind die positionsabhängigen Gegeninduktionsspannungen (BEMF-Spannungen), die bei Rotation des Rotors im Betrieb durch die Rotormagnetanordnung 242 des Rotors 240 in den Motorspulen 221 des Stators 220 erzeugt werden, bestimmbar. Die Gegeninduktionsspannungen werden unter anderem zur Bestimmung der Gegeninduktionskonstante KV ermittelt, aus der die Drehmomentkonstante kM ermittelt wird. Diese kann dann für die Bestimmung des Rotordrehmoments unter Verwendung der gemessenen Phasenströme genutzt werden.

Das Prinzip dieser Bestimmung der Gegeninduktionsspannung soll anhand der in FIG. 2 gezeigten Schaltskizze erläutert werden. Die Schaltskizze zeigt beispielhaft einen Motor 210 mit drei Motorphasen u, v, w. Allgemeiner kann eine Mehrzahl von Motorphasen, umfassend jeweils mindestens eine der Mehrzahl von Motorspulen 221, vorgesehen sein.

Die Motorphasen u, v, w werden durch ihre Induktivitäten $L_u$, $L_v$, $L_w$ und ihre Widerstände $R_u$, $R_v$, $R_w$ dargestellt, an denen die jeweilige Spannungen $U_{L,u}$, $U_{R,u}$, $U_{L,v}$, $U_{R,v}$, $U_{L,w}$, $U_{R,w}$ abfallen. Jeweilige induzierte Spannungen werden als $U_{u,\,ind}$, $U_{v,\,ind}$, $U_{w,\,ind}$ bezeichnet. Die Widerstände $R_u$, $R_v$, $R_w$ entsprechen dabei den Widerständen jeweiliger Anordnungen, die jeweils eine der Mehrzahl von Motorspulen 221 und eine zum Verbinden der jeweiligen Motorspule 221 mit der Steuereinheit 300 eingerichtete Zuleitung umfassen (Phasenwiderstände).

Als Komponenten des Messsignals sind die durch die Motorphasen u, v, w insgesamt fließenden Ströme $I_u$, $I_v$, $I_w$ sowie die an den Motorphasen u, v, w insgesamt anliegenden Spannungen $U_u$, $U_v$, $U_w$ erfassbar. Es gilt:

$$I_u R_u + U_u + \frac{dI_u}{dt} L_u + U_{u,ind} - I_v R_v - U_v - \frac{dI_v}{dt} L_v - U_{v,ind} = 0$$

$$I_v R_v + U_v + \frac{dI_v}{dt} L_v + U_{v,ind} - I_w R_w - U_w - \frac{dI_w}{dt} L_w - U_{w,ind} = 0$$

$$I_w R_w + U_w + \frac{dI_w}{dt} L_w + U_{w,ind} - I_u R_u - U_u - \frac{dI_u}{dt} L_u - U_{u,ind} = 0$$

(Gleichungen 1).

Bei bekannten Selbstinduktivitäten $L_u$, $L_v$, $L_w$ und Phasenwiderständen $R_u$, $R_v$, $R_w$ können BEMF-Spannungen (Gegeninduktionsspannungen durch Rotormagnete) $U_{BEMF1}$, $U_{BEMF2}$, $U_{BEMF3}$ wie folgt berechnet werden:

$$U_{BEMF1} = U_{u,ind} - U_{v,ind}$$

$$U_{BEMF2} = U_{v,ind} - U_{w,ind}$$

$$U_{BEMF3} = U_{w,ind} - U_{u,ind}$$

(Gleichungen 2).

Die magnetische Kopplung zwischen den Spulen kann mit weiteren Gegeninduktionstermen ( (dIv/dt) * Luv für Induktion von Phase v in Phase u) berücksichtigt werden. Die Statorspulen vieler Statorgeometrien können allerdings so symmetrisch angeordnet und verschaltet werden, dass die Gegeninduktionsterme sich gegenseitig aufheben, daher wurden diese Terme in Gleichung 1 eliminiert.

Aus den messbaren Gegeninduktionsspannungen und der ebenfalls erfassbaren Drehzahl des Rotors lässt sich der Gegeninduktionskoeffizienten $k_v$ und damit die Drehmomentkonstante $k_M$ bestimmen. Aus der Drehmomentkonstante $k_M$ und den Phasenströmen lässt sich dann das Drehmoment bestimmen und aus diesem und der Drehzahl der Durchfluss und/oder der Differenzdruck der Pumpe in erster Näherung schätzen.

Für viele Anwendungen reicht eine solche Bestimmung der Gegeninduktionsspannung aus. Für besonders genaue und vor allem auch gegenüber Temperaturänderungen unempfindliche Bestimmung der Gegeninduktionsspannung sowie eines Gegeninduktionskoeffizienten $k_v$ und damit der Drehmomentkonstanten $k_M$ kann es sinnvoll sein, auch die ohmschen Widerstände $R_u$, $R_v$ und $R_w$ im Betrieb laufend zu bestimmen, um die Gegeninduktionsspannung mit einer ausreichenden Genauigkeit bestimmen zu können. Sodann ist es sinnvoll, im Betrieb wiederholt, insbesondere regelmäßig, beispielsweise in gleichbleibenden zeitlichen Abständen oder auch ununterbrochen in aneinander angrenzenden Zeitintervallen den Gegeninduktionskoeffizienten kv zu bestimmen, um Störgrößen wie beispielsweise eine sich ändernde Rotorposition laufend zu berücksichtigen.

Im Folgenden soll zunächst beschrieben werden, wie die ohmschen Widerstände $R_u$, $R_v$ und $R_w$ im Betrieb laufend bestimmt werden. In einigen Fällen ist dies zur Steigerung der Messgenauigkeit für die Messung der Gegeninduktionsspannung nützlich. In anderen Fällen kann es auch möglich sein, auf die Bestimmung, zumindest auf eine laufende Bestimmung der Widerstände $R_u$, $R_v$ und $R_w$ zu verzichten, wenn beispielsweise im Betrieb eine gleichbleibende Temperatur der Anordnung, insbesondere der Zuleitungen, angenommen werden kann.

Weiter unten wird dann auf eine möglichst genaue Methode zur Ermittlung des Durchflusses und/oder des Differenzdrucks der Rotationsfluidpumpe aufgrund genauer Messung der elektrischen Größen eingegangen.

Auf der Grundlage der BEMF-Spannungen kann eine rotatorische Position (Drehwinkel) des Rotors 240 bezüglich der Rotationsachse 500 berechnet werden. Dazu werden die drei BEMF-Spannungen mittels Clark-Transformation in eine zweidimensionale Darstellung überführt und der Drehwinkel unter Verwendung der Arkustangensfunktion berechnet. Diese Berechnung ist also von $L_u$, $L_v$, $L_w$, $R_u$, $R_v$, $R_w$, $I_u$, $I_v$, $I_w$, $U_u$, $U_v$, $U_w$ abhängig.

Hierbei sind die Spannungs- und Strommessungen mit nur geringen Fehlern behaftet. Die Induktivitäten sind im Beispiel (Luftspulen) näherungsweise temperaturunabhängig und im für die Regelung relevanten Frequenzbereich nahezu konstant. Eine deutliche Temperaturabhängigkeit weist dagegen der Phasenwiderstand R (also $R_u$, $R_v$, $R_w$) auf:

$$R = R_{20}(1 + \alpha_{Cu}\Delta T), \qquad mit\ \alpha_{Cu} = 3.93 \cdot 10^{-3} \quad,$$

(Gleichung 3).

wobei $R_{20}$ der Widerstand bei einer Bezugstemperatur von 20 °C, $\Delta T$ eine Temperaturdifferenz und $\alpha_{Cu}$ ein material-abhängiger Temperaturkoeffizient (hier für Spulenwindungen und Zuleitungsadern aus Kupfer) ist. Beispielsweise ändern 10 K Temperaturunterschied den Widerstand um ca. 4 %. Auch andere Faktoren (z. B. Drift, Korrosion, Bruch einer redundanten Driveline-Ader, thermische Einflüsse, variable Übergangswiderstände in Steckern) können den Phasenwiderstand beeinflussen, der somit als zeitlich veränderlich anzusehen ist und zum Zweck der Regelung im laufenden Betrieb der Pumpe bestimmt werden soll.

Die Steuereinheit 300 ist dementsprechend eingerichtet zum Bestimmen des jeweiligen Phasenwiderstands mittels der hier vorgeschlagenen Verfahren.

Insbesondere ist die Steuereinheit 300 eingerichtet zum

Erzeugen eines Steuersignals für eine jeweilige der Mehrzahl von Motorspulen 221, wobei das Steuersignal mit einem Modulationssignal beaufschlagt wird,

Demodulieren des Messsignals zum Bestimmen eines veränderlichen charakteristischen elektrischen Widerstands einer elektrischen Anordnung (Phasenwiderstand), umfassend die jeweilige der Mehrzahl von Motorspulen und eine jeweilige Zuleitung zum Verbinden der jeweiligen der Mehrzahl von Motorspulen 221 mit der Steuereinheit 300.

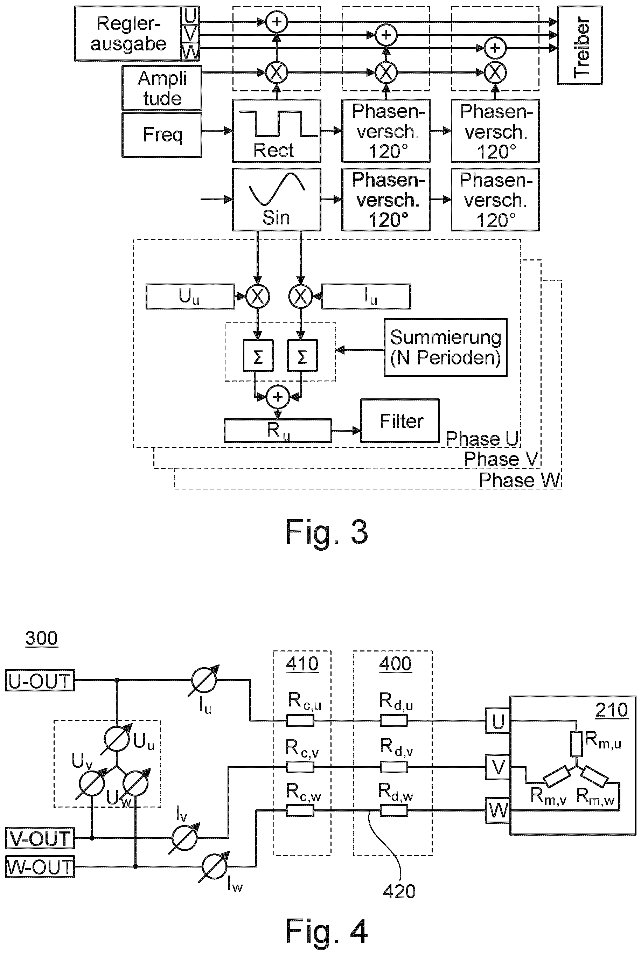

Ein Beispiel des vorbeschriebenen Verfahrens ist illustriert in FIG. 3. Die Bestimmung der Phasenwiderstände ist hierbei als kontinuierliche Hintergrundmessung im Betrieb der Pumpe implementiert. Das Steuersignal entspricht dabei einer jeweiligen Reglerausgabe für die Phasen u, v, w. Die dem Steuersignal entsprechende Spannung wird durch Pulsweitenmodulation (PWM) als PWM-Signal bereitgestellt.

Zur Messung des Phasenwiderstands, $R_p$, zu einer gegebenen Motorphase (p = u, v, w) ist die Steuereinheit 300 dazu eingerichtet, das Steuersignal mit dem Modulationssignal durch Variation der Pulsbreitenmodulation, hier durch Aufaddieren einer Signalform, insbesondere eines Rechtecksignals, auf das PWM-Signal zu beaufschlagen. Dies kann nacheinander für jede Phase oder phasenversetzt (um 120 °) erfolgen, wie in FIG. 3 gezeigt. Mittels eines Treibers werden die entsprechenden Motorspulen 221 mit dem so modifizierten Steuersignal beaufschlagt.

Die Amplitude des Modulationssignals ist dabei variabel und wird vorzugsweise so gewählt, dass Störungen des Motorbetriebs vermieden bzw. minimiert werden (bsp. als kleinste Auflösung der PWM). Die Frequenz des Modulationssignals wird ebenfalls vorzugsweise so gewählt, dass Störungen vermieden werden, beispielsweise im Hinblick auf die Drehzahl der Pumpe und/oder eine Herzfrequenz. Eine Modulationsfrequenz des Modulationssignals kann beispielsweise weniger als eine Drehfelddrehzahl, vorzugsweise weniger als 50 Hz, betragen. Beispielhaft kann etwa eine Frequenz von 10 Hz gewählt werden. Die Drehfelddrehzahl 240 kann beispielweise im Bereich von 0,1 bis 1 kHz liegen.

Die Steuereinheit 300 ist dazu eingerichtet, einen durch das Beaufschlagen des Steuersignals mit dem Modulationssignal verursachten Modulationsanteil des Messsignals mittels Demodulation zu erfassen und aus dem demodulierten Signal den Phasenwiderstand zu bestimmen.

Das Messsignal ($I_p$, $U_p$ mit p = u, v, w) wird dazu im jeweiligen Modulationszustand an dem jeweils mit dem Modulationssignal beaufschlagten Satz von Motorphasen 221 abgegriffen. Die einzelnen Messpunkte des Messsignals können mit dem Modulationssignal oder einem Sinus der jeweiligen Phase gewichtet werden. Zusätzlich oder alternativ kann zur Gewichtung eine Fensterfunktion angewendet werden, um mehr Frequenzanteile für die Strom- und Spannungsmessung zu erfassen, wodurch ein besserer Signal-Rausch-Abstand erzielbar sein kann.

Das (ggf. gewichtete) Messsignal wird über N Perioden summiert (also kumuliert, alternativ gemittelt). Die N Perioden entsprechen dabei beispielsweise einem Intervall von mindestens 1 s und/oder höchstens 10 s. Das Intervall kann wie erwähnt an die momentanen Anforderungen angepasst werden; beispielsweise kann eine schnelle, kurzzeitige Messung mit reduziertem Intervall bei erhöhter Modulationsamplitude durchgeführt werden.

Aus dem kumulierten Signal für die Ströme und Spannungen ($I_p$, $U_p$) erfolgt die Berechnung des Phasenwiderstands. Ein zusätzlicher Filter kann angewandt werden, um die erhaltenen Werte zu glätten oder gestörte Werte zu entfernen.

FIG. 4 illustriert beispielhafte Komponenten des Pumpensystems 100 für das in FIG. 3 illustrierte Verfahren. Motor 210 und Steuereinheit 300 sind mittels der Driveline 400 und einer lösbaren Steckverbindung 410 miteinander verbunden. Die Driveline 400 in Verbindung mit der Steckverbindung 410 umfasst Zuleitungen zum Verbinden jeder der Motorspulen 221 mit der Steuereinheit 300. Jede der Zuleitungen umfasst eine oder mehrere leitende Adern 420 und einen Kontakt, hier einen Steckkontakt der Steckverbindung 410, zum Verbinden der Ader 420 mit der Steuereinheit 300. Die Kontakte können alternativ fest ausgeführt sein. Der Phasenwiderstand jeder Phase p = u, v, w umfasst einen Widerstand $R_{m,p}$ der jeweiligen Motorspule bzw. Motorspulen, einen Widerstand $R_{d,p}$ der jeweiligen Ader bzw. Adern der Driveline und einen Widerstand $R_{c,p}$ des jeweiligen Kontakts. Die Steuereinheit umfasst jeweilige Mittel zum Erfassen der an den jeweiligen Anordnungen abfallenden Spannungen $U_p$ und der dadurch fließenden Ströme $I_p$ (jeweilige Schaltzeichen mit Pfeil im Kreis).

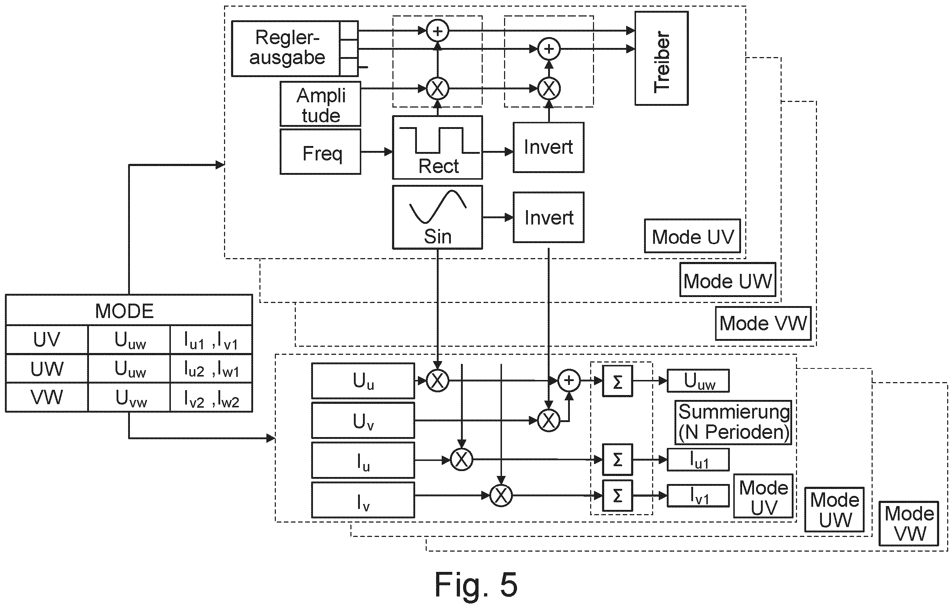

FIG. 5 illustriert ein weiteres Beispiel des vorgeschlagenen Verfahrens. Ausgangspunkt ist die Überlegung, dass bei dem Beispiel nach FIG. 3/4 eine virtuelle Spannungsmessung (mit virtuellem Sternpunkt 310 der Spannungsmessung wie in FIG. 4 gezeigt) erfolgt, die nur dann genau ist, wenn die Phasenwiderstände aller Phasen näherungsweise gleich sind. Eine signifikante Abweichung eines Phasenwiderstands von den übrigen führt zu einer Verfälschung der Werte an den anderen Phasen. Um dennoch eine genaue Messung durchführen zu können, kann vorgesehen werden, die Sternpunkte galvanisch zu verbinden. Da diese Lösung einen zusätzlichen Leiter erfordert, wird die nachfolgend beschriebene alternative Verfahrensvariante bereitgestellt.

Hierbei wird ein differentielles Messverfahren angewandt. Die Modulation findet dabei beispielsweise gegenphasig an zwei Phasen statt, während die dritte Phase nicht angeregt wird. Die Steuereinheit 300 ist also dazu eingerichtet, nacheinander eine Mehrzahl von Modulationszuständen (im Beispiel nach FIG. 5 als Mode uv/uw/vw bezeichnet) vorzugeben, wobei in jedem Modulationszustand ein jeweiliger Satz von Motorphasen 221 gegenphasig oder phasenverschoben mit dem Modulationssignal beaufschlagt wird.

Synchron zum jeweiligen Modulationszustand wird eine Differenz der an den entsprechenden Motorspulen anliegenden Spannungen sowie der jeweiligen Ströme mittels eines Sinusdetektors der Steuereinheit 300 erfasst. Die Messung wird wie oben über N Perioden wiederholt und kumuliert, anschließend wird das Phasenpaar gewechselt. Insgesamt werden so im vorliegenden Beispiel also in drei Messschritten (entsprechend den drei Modulationszuständen) drei Differenzspannungen $U_{uv}$, $U_{uw}$, $U_{vw}$ und sechs Ströme $I_{u1}$. $I_{v1}$, $I_{w1}$, $I_{u2}$, $I_{v2}$, $I_{v2}$ erfasst. Diese erfüllen die folgenden Gleichungen:

$$U_{uv} = R_u \cdot I_{u1} + R_v \cdot I_{v1}$$
$$U_{uw} = R_u \cdot I_{u2} + R_w \cdot I_{w1}$$
$$U_{vw} = R_v \cdot I_{v2} + R_w \cdot I_{w2}$$

(Gleichungen 4).

Dabei ist $U_{uv}$ die erfasste Amplitude der Spannungsdifferenz zwischen Phase u und v, $U_{uw}$ und $U_{vw}$ analog. $I_{u1}$ ist die erfasste Amplitude des Stroms in Phase v während des uv-Modulationszustands, $I_{v1}$, $I_{w1}$, $I_{u2}$, $I_{v2}$, $I_{v2}$ analog.

Hieraus können die Phasenwiderstände der Phasen u, v, w berechnet werden:

$$R_W = \frac{U_{vw} \cdot I_{v1} \cdot I_{u2} + U_{uw} \cdot I_{u1} \cdot I_{v2} - U_{uv} \cdot I_{u2} \cdot I_{v2}}{I_{w1} \cdot I_{u1} \cdot I_{v2} + I_{w2} \cdot I_{v1} \cdot I_{u2}}$$

$$R_v = \frac{U_{vw}}{I_{v2}} - \frac{R_W \cdot I_{w2}}{I_{v2}}$$

$$R_u = \frac{U_{uw}}{I_{u2}} - \frac{R_W \cdot I_{w1}}{I_{u2}}$$

(Gleichungen 5).

Das differentielle Messverfahren nach FIG. 5 zeichnet sich durch eine besonders hohe Genauigkeit aus, da eine Änderung eines Phasenwiderstands wie erwähnt nicht an die Messung der der übrigen Phasenwiderstände gekoppelt ist.

Im Folgenden werden noch einige alternative Varianten der beschriebenen Messverfahren beschrieben. Bei dem vorbeschriebenen differentiellen Verfahren wird jeweils eine (dritte) Motorphase nicht angeregt und dementsprechend kein dazu gehöriges Messsignal erfasst. Es ist zusätzlich möglich, auch die dritte Phase aktiv mit einem Strom zu beaufschlagen und das dem Strom in der dritten Phase entsprechende Messsignal zu erfassen. Dann kann das differentielle Messverfahren mit zwei statt der oben beschriebenen drei Schritte durchgeführt werden, wobei in beiden Schritten die jeweilige dritte Phase mit unterschiedlichen Strömen beaufschlagt wird.

Die Phasenwiderstände können dann auf Grundlage des folgenden Gleichungssystems berechnet werden:

$$\begin{pmatrix} U_{vw1} \\ U_{uw1} \\ U_{vw2} \end{pmatrix} = \begin{pmatrix} R_v & R_w & 0 & 0 \\ -R_u & R_w - R_u & 0 & 0 \\ 0 & 0 & R_v & R_w \end{pmatrix} \begin{pmatrix} I_{v1} \\ I_{w1} \\ I_{v2} \\ I_{w2} \end{pmatrix}$$

(Gleichung 6).

Dabei ist $U_{vw1}$ die erfasste Amplitude der Spannungsdifferenz zwischen Phase v und w während des ersten Messschritts, $U_{uw1}$ und $U_{vw2}$ analog. $I_{w2}$ ist die erfasste Amplitude des Stroms in Phase w während des zweiten Messschritts, $I_{v1}$, $I_{w1}$, $I_{v2}$ analog.

Es ergibt sich für die Phasenwiderstände:

$$R_u = \frac{-I_{v1}I_{w1}U_{vw2} - I_{v1}I_{w2}U_{uw1} + I_{v2}I_{w1}U_{uw1} - I_{v2}I_{w1}U_{vw1}}{I_{v1}{}^2 I_{w2} - I_{v1}I_{v2}I_{w1} + I_{v1}I_{w1}I_{w2} - I_{w1}{}^2 I_{v2}}$$

$$R_v = \frac{-I_{w1}U_{uw1} + I_{w2}U_{vw1}}{I_{v1}I_{w2} - I_{v2}I_{w1}}$$

$$R_w = \frac{I_{v1}U_{uw} - I_{v2}U_{vw1}}{I_{v1}I_{w2} - I_{v2}I_{w1}}$$

(Gleichungen 7).

Bei einer Vektorregelung der Drehung des Rotors mit zweidimensionalem dq-System können auch die d- und q-Komponenten direkt moduliert werden. In diesem Fall entsteht ein mit der Drehung gemischtes Messsignal der Spannungsdifferenzen und Ströme. Dieses kann direkt mit entsprechenden Detektoren erfasst und/oder vor dem Erfassen mit der Drehung entmischt werden. Die führt dazu, dass das eingeprägte Messignal nur entweder dem Antriebsmoment oder der Lagerkraft auftritt und somit nur einen Regler störend beeinflusst.

Unabhängig von dem speziell gewählten Messverfahren kann anstelle der erwähnten Rechtecksmodulation auch eine andere Signalform vorgesehen sein. Hier kommen etwa Sinus, Rauschen oder Pseudozufallssequenzen, beispielsweise Gold-Codes, in Frage. Alle Messverfahren sind hier für drei Motorphasen illustriert, lassen sich aber in offensichtlicher Weise auf andere Zahlen von Motorphasen verallgemeinern.

Es kann vorgesehen sein, dass die Steuereinheit 300 zum alternativen Durchführen beider Messverfahren, also der differentiellen Messung entsprechend FIG. 5 und der Einzelphasenmessung nach FIG. 3, eingerichtet ist. Die Steuereinheit 300 kann insbesondere dazu eingerichtet sein, bei einem Ausfall eines Detektors für das Messsignal einer Phase von der differentiellen Messung auf die Einzelphasenmessung zu wechseln. Auf diese Weise ist eine Messung auch bei einem solchen Ausfall weiterhin möglich. Die Steuereinheit kann in diesem Fall eine Warnung ausgeben, so dass die Steuereinheit gewartet oder ausgetauscht werden kann.

Unabhängig von dem speziell gewählten Messverfahren ist die Steuereinheit 300 bevorzugt dazu eingerichtet, die Rotation des Rotors 240 sowie zumindest eine translatorische Position des Rotors 240 (insbesondere entlang der Rotationsachse) basierend auf dem Messsignal und einem Modell wenigstens eines Teils der Rotationsfluidpumpe 200 zu erfassen und zu regeln, wobei das Modell den Phasenwiderstand umfasst. Zusätzlich können translatorische und/oder rotatorische Positionen in weiteren Freiheitsgraden regelbar sein.

Die Steuereinheit 300 ist dazu eingerichtet, die Phasenwiderstände wiederholt für jeweils ein erstes Messintervall durch Demodulieren des mindestens einen Messsignals gemäß den oben beschriebenen Verfahren zu bestimmen. Die Steuereinheit kann ferner dazu eingerichtet sein, eine Änderung des Phasenwiderstandes über ein zweites Messintervall (insbesondere ebenfalls wiederholt für jeweils ein zweites Messintervall) basierend auf einer Temperaturänderung in mindestens einer der Motorspulen zu bestimmen. Das zweite Messintervall ist dabei kürzer als das erste Messintervall. Somit können Messungen über das erste Messintervall durch Demodulieren und über das zweite Messintervall basierend auf der Temperaturänderung miteinander kombiniert werden, wodurch eine Bestimmung des Phasenwiderstands bzw. seiner Änderungen mit guter Genauigkeit über sowohl kürzere als auch längere Zeitskalen möglich ist. Die Steuereinheit 300 ist zum Zweck der Bestimmung mittels Temperaturänderung eingerichtet zum:

Bestimmen, basierend auf dem mindestens einen Messsignal und einem thermischen Modell, einer Änderung einer Temperatur wenigstens eines Teils der Rotationsfluidpumpe 200, umfassend die mindestens eine der Mehrzahl von Motorspulen 221 (im Folgenden kurz Motortemperatur), über das zweite Messintervall; und

Bestimmen, basierend auf der bestimmten Änderung der Motortemperatur, eines Wertes oder einer Änderung des Phasenwiderstands für die mindestens eine der Mehrzahl von Motorspulen 221.

Somit ist eine schnelle Schätzung des Phasenwiderstands bei Temperaturänderungen aufgrund kurzzeitig eingebrachter Leistung möglich. Eine solche Erwärmung erfolgt in Bruchteilen einer Sekunde, entsprechend kurz wird das zweite Messintervall gewählt.

Als thermisches Modell kann ein einfaches Modell gewählt werden, beispielsweise wie folgt, basierend auf einer thermischen Kapazität $C_{th}$:

$$C_{th}\frac{dT}{dt} = \dot{Q} = P - E$$

(Gleichung 8).

Dabei ist Q die eingebrachte Wärme, P die eingebrachte Leistung, E die an die Umgebung abgegebene Leistung und T die Motortemperatur. Die Steuereinheit 300 ist also dazu eingerichtet sein, die Änderung der Motortemperatur (und damit letztlich die Änderung des Phasenwiderstands) basierend auf einem auf Grundlage des Messsignals bestimmten Leistungseintrag in die Rotationsfluidpumpe 200 und basierend auf einer geschätzten thermischen Leistungsabgabe der Rotationsfluidpumpe 200 zu bestimmen. Die eingebrachte Leistung P kann präzise aufgrund der Strom- und Spannungsmessung berechnet werden. Die abgegebene Leistung E wird dagegen geschätzt, etwa durch folgende Näherung:

$$E = \frac{1}{R_k}(T - T_e)$$

(Gleichung 9).

Dabei ist $R_k$ ein thermischer Kontaktwiderstand (darstellbar in Einheiten von K/W), $T_e$ die Temperatur eines Kontaktmaterials. In einer Variante kann auch die an den Rotor übertragene Leistung in der abgegebenen Leistung E berücksichtigt werden.

Im Betrieb der Pumpe besteht normalerweise ein Gleichgewicht zwischen Motortemperatur und Temperatur des Kontaktmaterials. Wird plötzlich die Leistung P sehr groß, kommt es zu einer schnellen Änderung der Motortemperatur. $T_e$ wird sich demgegenüber in aller Regel deutlich langsamer ändern, im Fall einer implantierten Blutpumpe insbesondere aufgrund der größeren Wärmekapazität und aktive Kühlung durch das Blut. Diese Änderung hängt von zahlreichen Einflussfaktoren (einschließlich Strömungsbedingungen in der Pumpe sowie Blut- und Körpertemperatur) ab und kann daher nicht genau geschätzt werden. In der vorbeschriebenen (langsamen) Bestimmung des Phasenwiderstands über das erste Messintervall durch Modulation ist jedoch näherungsweise die Information über ein mittleres $T_e$ enthalten, da mit dem Widerstand auch T genau bekannt ist. Basierend auf der Annahme, dass $T_e$ konstant ist, kann das Modell der thermischen Kapazität linearisiert werden.

Die Änderung $\Delta R$ des Phasenwiderstands R kann schließlich geschätzt werden als:

$$\Delta R = R_0 \alpha_{Cu} \Delta T, \quad mit \ \Delta T = \frac{R_k}{(1 + R_k C_{th} s)} UI$$

(Gleichung 10).

Dabei ist $R_0$ der elektrische Widerstand bei der Ausgangstemperatur, $\Delta T$ die geschätzte Temperaturdifferenz und $\alpha_{Cu}$ ein materialabhängiger Temperaturkoeffizient wie oben definiert.

Die Messungen über das erste Messintervall durch Demodulieren und über das zweite Messintervall basierend auf der Temperaturänderung können etwa folgendermaßen miteinander kombiniert werden:

$$R = R_s + \Delta R - \Delta R_{lp}$$

(Gleichung 11).

Dabei ist R der Phasenwiderstand, $R_s$ der mittels Modulation/Demodulation ("Hintergrundmessung") geschätzte Wert (bestimmt mit dem langsameren Takt entsprechend dem Intervall $T_l$), $\Delta R$ die aufgrund der Temperaturänderung geschätzte Differenz (bestimmt mit dem schnelleren Takt entsprechend dem Intervall $T_s$, beispielsweise dem Takt der Regelung), $\Delta R_{lp}$ der zwischen dem letzten und vorletzten Update von $R_s$ gemittelte Wert von $\Delta R$.

Gleichung 11 wurde aufgrund der folgenden Überlegungen gewählt. Schnelle Widerstandsänderungen durch Leistungseintrag sollten im Gesamtwiderstand sofort berücksichtigt werden. Allerdings wirkt sich die Erwärmung auch auf die Hintergrundmessung aus. Daher wird bei jedem Update der Mittelwert der durch Leistung erzeugten Widerstandsänderung abgezogen, da dieser in dem neuen Wert $R_s$ der Hintergrundmessung enthalten ist.

Die Steuereinheit 300 kann zusätzlich dazu eingerichtet sein, basierend auf dem Phasenwiderstand einen Verbindungszustand zwischen der Rotationsfluidpumpe 200 und der Steuereinheit 300 und/oder einen Defektzustand der Rotationsfluidpumpe 200 und/oder der Driveline 400 zu erfassen (wie weiter oben beschrieben aufgrund von entsprechenden Änderungen des Phasenwiderstands). Für das Erfassen des Verbindungszustands kann etwa vorgesehen sein, bei getrennter Verbindung eine Widerstandsmessung mit hoher Amplitude und geringer Integrationszeit durchzuführen, wodurch eine schnelle Messung möglich ist. Bei Herstellen der Verbindung (erfassbar als sprunghafte Widerstandsänderung) kann dann entsprechend der obigen Ausführungen zu diesen Parametern die Amplitude gesenkt und die Integrationsdauer erhöht werden. Bei Entfernen der Pumpe wird erneut in den schnellen Modus gewechselt.

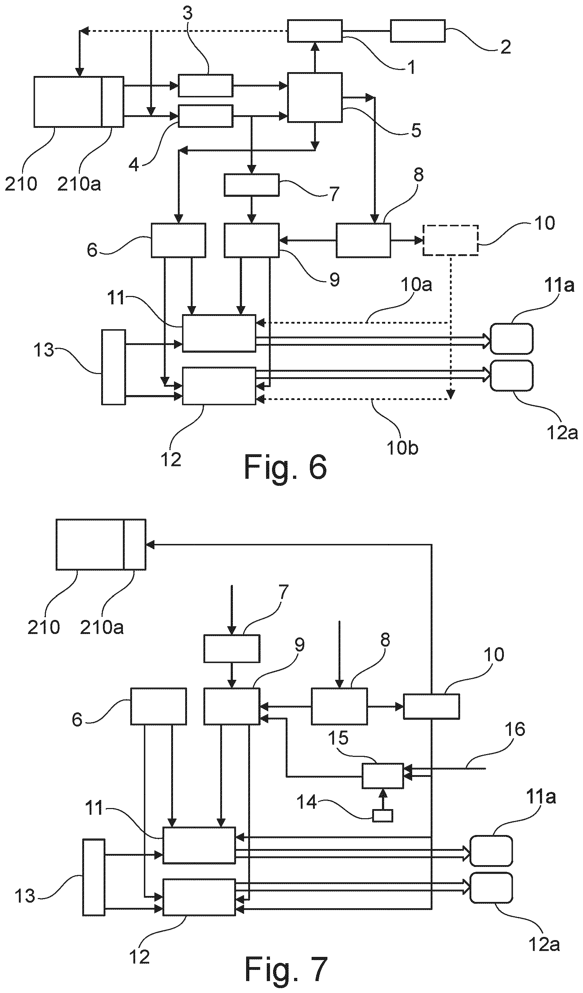

Im Folgenden wird das Verfahren zur Bestimmung der Durchflussrate und/oder eines Differenzdrucks der Rotationsfluidpumpe aus erfassten elektrischen Messgrößen anhand der Figuren 6 und 7 beschrieben.

Eine wesentliche Größe zur Bestimmung der Durchflussrate und/oder eines Differenzdrucks einer Rotationsfluidpumpe ist das Drehmoment des Rotors, das auf das zu fördernde Fluid, insbesondere die Flüssigkeit wirkt. Dieses kann aus der Motorstromstärke bestimmt werden nach der Gleichung

$$M = I * K_M$$

als Produkt aus der Drehmomentstromstärke $I = I_{q\_messung}$ und der Drehmomentkonstanten $K_M$. $I_{q\_messung}$ kann aus den Phasenströmen und dem Rotorwinkel beispielsweise mit der Clarke-Park-Transformation bestimmt werden. Es ist aus den Grundlagen der Elektrotechnik bekannt, dass die Drehmomentkonstante $K_M$ gleich der Gegeninduktionskonstante $K_V$ ist. Diese Gegeninduktionskonstante $K_V$ ist aus der Messung der Drehzahl des Motors und der Gegeninduktionsspannung ermittelbar als Quotient aus der Induktionsspannung $U_{Ind}$ des Motors und der Winkelgeschwindigkeit $\omega$ des Rotors nach der Gleichung:

$$K_V = U_{Ind} / \omega$$

Somit läßt sich das Drehmoment des Pumpenrotors berechnen aus:

$$M = I * U_{Ind} / \omega.$$

Die Gegeninduktionsspannung kann dabei je nach den Genauigkeitsanforderungen und den Umgebungsbedingungen mit oder ohne die oben detailliert beschriebene Berücksichtigung der Zuleitungswiderstände bestimmt werden. Die Drehzahl $\omega$ kann beispielsweise auch aus dem zeitlichen Verlauf der Gegeninduktionsspannung bestimmt werden.

Die beiden Konstanten $K_M$ und $K_V$ stimmen in ihrer Abhängigkeit von der Rotorposition (beispielsweise konkret der axialen Position des Rotors in seinem Lager), der Magnetstärke der Rotormagneten, der Alterung von Materialien, Bauteilstreuung und der Temperatur weitestgehend überein, so dass Änderungen dieser Größen bei der Bestimmung des Drehmomentes ohne weitere Maßnahmen kompensiert sind.

Für eine möglichst genaue Bestimmung der Durchflussrate und/oder eines Differenzdrucks kann es jedoch nützlich sein, zusätzlich die hydrodynamische Effizienz der Pumpe zu berücksichtigen, die von der Rotorposition abhängig ist, sowie parasitäre Bremsmomente, die unabhängig von dem zu fördernden Fluid auf den Rotor wirken, und die Viskosität des zu fördernden Fluids, insbesondere dann, wenn es sich um eine Flüssigkeit wie beispielsweise Blut handelt.

Da die ermittelten Konstanten $K_M$ und $K_V$ eine deutliche Abhängigkeit von der Rotorposition aufweisen, kann nach Bestimmung dieser Konstanten aus diesen die Rotorposition sehr genau bestimmt werden. Die Abhängigkeit der beiden Konstanten von der Rotorposition ist beispielsweise deutlich stärker als die Abhängigkeit der Messgrößen von bisher zur Rotorpositionsmessung eingesetzten Wirbelstromsensoren von der Rotorposition. Aus diesem Grund gelingt die Bestimmung der Rotorposition mit den Konstanten $K_M$ und $K_V$ sehr genau.

Parasitäre Bremsmomente auf den Rotor können dabei beispielsweise durch die von den Rotormagneten erzeugten Wirbelstrom-Bremseffekte im Material des Pumpengehäuses oder anderer Pumpenbauteile entstehen. Nach Ermittlung der parasitären Bremsmomente können diese bei der Ermittlung des auf das Fluid wirkenden Drehmomentes berücksichtigt werden.

Die ermittelten Werte des Drehmomentes und der Drehzahl werden einer Zuordnungseinheit zugeführt, die eine Durchflussrate und/oder einen Differenzdruck der Pumpe bestimmt. Die Zuordnungseinheit weist ein Zuordnungsmodell auf, das beispielsweise als selbstlernendes Modell, insbesondere als trainiertes neuronales Netz oder einfach als Kennlinienfeld beziehungsweise als Look-up- Tabelle ausgebildet sein kann. Es kann auch einen sogenannten "Beobachter" enthalten, der die geltenden mathematischen Zusammenhänge zur Ermittlung der gewünschten Größen abbildet. Es können auch näherungsweise Abbildungsfunktionen an die Messgrößen angefittet werden, um eine Durchflussrate und/oder einen Differenzdruck zu schätzen.

Für eine Steigerung der Genauigkeit und Zuverlässigkeit der Schätzungen ist es vorteilhaft, bei der Ermittlung der Gegeninduktionsspannung nach der oben beschriebenen Methode den Leitungswiederstand laufend zu ermitteln und zu berücksichtigen, ebenso wie die Rotorposition, insbesondere in Axialrichtung, um die hydrodynamische Effizienz des Rotors oder genauer gesagt der Pumpengeometrie zu berücksichtigen. Die Rotorposition kann gesondert aus den Konstanten $k_V$ und $K_M$ ermittelt und in die Zuordnungseinheit eingegeben werden. Auch die parasitären Drehmomente, die durch Wirbelstromverluste entstehen können, können in dem Modell berücksichtigt werden, indem es auch mit diesen Größen trainiert wird oder diese zusätzlichen Abhängigkeiten, beispielsweise von der Temperatur und den Phasenströmen, in dem Modell berücksichtigt werden.

In der Figur 6 ist das Verfahren zur Bestimmung der Durchflussrate und/oder eines Differenzdrucks einer Rotationsfluidpumpe anhand von Prozessschritten schematisch dargestellt.

Der Elektromotor 210 der Pumpe, die eine implantierbare oder extrakorporale Blutpumpe sein kann, wird mit seinen Antriebswicklungen durch einen Motortreiber 1 mit elektrischen Antriebssignalen versorgt. Zudem werden Magnetkräfte durch eine Lagerregelung 210a in Magnetlagern des Motors durch Lagerströme erzeugt. Die elektrische Energie bezieht der Motortreiber 1 dabei von einer Energiequelle 2, die als Akku/wiederaufladbare Batterie ausgebildet sein kann. In dem Spannungsmessmodul 3 werden die Phasenspannungen gemessen und in dem Strommessmodul 4 werden die Phasenströme gemessen.

In dem Back- EMF- Modul 5 wird aus dem zeitlichen Verlauf der Phasenströme und ihrer zeitlichen Ableitung sowie den gemessenen Phasenspannungen die Gegeninduktionsspannung zeitabhängig bestimmt. In diesem Schritt kann durch die weiter oben beschriebene Aufmodulation von elektrischen Signalen auf die Antriebssignale des Motors und die beschriebenen Messungen der ohmsche Widerstand der Zuleitungen bestimmt und bei der genauen Ermittlung der Gegeninduktionsspannungen berücksichtigt werden. Zudem kann aus dem zeitlichen Verlauf der Gegeninduktionsspannung sehr genau die Drehzahl des Motors und der absolute Drehwinkel bestimmt werden. Aus den ermittelten Werten der Gegeninduktionsspannung und der Drehzahl wird in dem Modul 8 die Gegeninduktionskonstante $K_V$ und damit auch $K_M$ bestimmt. Die Drehzahl des Motors wird auch in dem Modul 6 bereitgestellt.

In dem Modul 7 werden die gemessenen Phasenstromstärken bereitgestellt. Diese werden in dem Modul 9 zur Berechnung des erzeugten elektrischen Gesamtdrehmomentes des Motors weiter verarbeitet. Dazu wird dem Modul 9 außer der Stromstärke auch aus dem Modul 8 die ermittelte Drehmomentkonstante $K_M = K_V$ übermittelt.

Damit stehen in den Modulen 6 und 9 die Drehzahl des Motors und das Gesamtdrehmoment für weitere Berechnungen zur Verfügung.

Es ist dabei zu bedenken, dass das elektrische Drehmoment des Rotors nicht vollständig auf das zu fördernde Fluid übertragen wird, da es noch weitere Bremsmomente gibt, die den Rotor mechanisch bremsen, wie beispielsweise Wirbelstrombremseffekte, auf die weiter unten im Zusammenhang mit der Figur 7 noch eingegangen wird.

Die Drehzahl und das Drehmoment werden an die Berechnungsmodule 11 und 12 der Steuereinheit übermittelt, wobei das Modul 11 eine Durchflussrate und das Modul 12 einen Differenzdruck schätzen können.

Die Module 11 und 12 können als trainiertes selbstlernendes System, beispielsweise in der Form von neuronalen Netzen ausgebildet sein oder als Beobachter. Das Modul 11 gibt in der Ausgabeeinheit 11a eine Durchflussrate aus und das Modul 12 in der Ausgabeeinheit 12a einen Differenzdruck der Pumpe.

Die Bestimmung der Durchflussrate und des Differenzdrucks sind unter anderem auch von der Viskosität des zu fördernden Fluids abhängig. Aus diesem Grund kann die Viskosität in einem Modul 13 gemessen, eingegeben oder anderweitig bestimmt werden, so dass sie den Modulen 11 und 12 zur genaueren Bestimmung der Durchflussrate und/oder eines Differenzdrucks übermittelt werden kann.

Da die Abhängigkeiten von $K_V$ und $K_M$ jeweils von den Verhältnissen der Pumpe und der Temperatur sowie der Rotorposition gleich sind, werden Änderungen dieser Größen durch die Bestimmung von $K_M$ aus $K_V$ automatisch ausgeglichen.

Da jedoch eine sensible Abhängigkeit der beiden genannten Konstanten von der Rotorposition, insbesondere in Axialrichtung des Rotors, besteht, kann aus den Konstanten die Rotorposition, insbesondere in Axialrichtung, sensitiv und mit hoher Genauigkeit sowie langzeitstabil ermittelt werden. Der Schritt der Bestimmung der Axialposition des Rotors aus den Konstanten $K_M$ / $K_V$ ist mit 10 bezeichnet.

Ist die Axialposition des Rotors bestimmt, so kann sie in den Schritten 10a, 10b den Berechnungsmodulen 11 und 12 übermittelt werden, um die Durchflussrate und den Differenzdruck genauer zu ermitteln, da die hydrodynamische Effizienz des Rotors relativ stark von der Position, insbesondere der Axialposition des Rotors abhängt. Der Einfluss der verschiedenen Messgrößen auf die zu ermittelnde Durchflussrate und den Differenzdruck kann jeweils durch ein vorangehendes Training einer selbstlernenden Einheit oder durch hinterlegte Kennlinien, Tabellen oder Funktionen in den Modulen 11 und 12 berücksichtigt werden. Eine Bestimmung der Durchflussrate und des Differenzdrucks ist jedoch in erster Näherung auch ohne die Berücksichtigung beispielsweise der Rotorposition möglich.

Die genannten Module können jeweils als Bearbeitungsschritte in einem Ermittlungsprozess gestaltet werden, der mittels einer Computereinheit implementiert und durchgeführt werden kann.

In der Literatur ist die Definition von der Gegeninduktionskonstante kv nicht immer einheitlich. Kv kann, wie in der vorliegenden Anmeldung, als Induktionsspannung pro Winkelgeschwindigkeit definiert werden, aber auch der skalierte Kehrwert in tausend Umdrehungen pro Minute pro Induktionsspannung ist durchaus üblich. In beiden Fällen ist die Beziehung zu $K_m$ unterschiedlich, allerdings lässt sich km immer eindeutig Aus $K_V$ bestimmen.

In der Figur 7 sind zusätzlich zu den in der Figur 6 dargestellten Verarbeitungsstrukturen und Verarbeitungsschritten noch weitere Korrekturmöglichkeiten durch Berücksichtigung und Verarbeitung weiterer Störfaktoren gezeigt. Einige Module der Figur 6 sind nur der Übersichtlichkeit halber in der Figur 7 weggelassen, obwohl sie bei der Verarbeitung dort ebenso vorgesehen sind. Von zentraler Bedeutung ist das Modul 9 zur Bestimmung des Drehmomentes des Rotors aus den Phasenströmen. Zudem ist das Modul 8 wichtig, in dem die Gegeninduktionskonstante $k_V$ und damit die Drehmomentkonstante $k_M$ bestimmt wird. Diese Konstanten werden dem Modul 9, ebenso wie die gemessenen Phasenstromstärken, übermittelt.

Aus den genannten Konstanten wird auch laufend die Rotorposition in einem Modul 10 ermittelt. Die Rotorposition wird über den Übermittlungsweg 10a und 10b zur Berücksichtigung der hydrodynamischen Effizienz des Rotors oder der Pumpe an die Module 11 und 12 zur Ermittlung einer korrigierten Durchflussrate und eines Differenzdrucks übermittelt.

Zudem wird die genau ermittelte Axialposition und/oder eine Radialposition des Rotors an die Lagerregelung 210a der Magnetlager zur genauen Regelung der Rotorposition übermittelt.

Bei der Ermittlung des Drehmomentes in dem Modul 9 wird darauf abgezielt, das tatsächlich auf das zu fördernde Fluid wirkende Drehmoment zu bestimmen. Hierzu werden von dem theoretischen, elektrisch erzeugten Drehmoment, das aus den Phasenstromstären und der Drehmomentkonstanten berechnet wird, die Drehmomentverluste abgezogen, die durch parasitäre Bremsmomente infolge von Wirbelströmen und Hystereseverlusten entstehen. Dabei sind die Wirbelströme, die durch die Rotormagnete in metallischen Elementen in unmittelbarer Nähe, beispielsweise Teile des Pumpengehäuses, erzeugt werden, von der Drehzahl des Motors, der Rotorposition und der Temperatur der metallischen Elemente abhängig, in denen die Wirbelströme erzeugt werden. Aus diesem Grund wird für eine genaue Ermittlung der Bremsmomente mittels eines Temperatursensors in einem Modul 14 die Temperatur des Pumpengehäuses ermittelt und gemeinsam mit der Rotorposition und der in dem Modul 6 ermittelten Drehzahl des Rotors, repräsentiert durch den Pfeil 16, an das Modul 15 zur Ermittlung der Bremsmomente übermittelt. Das Bremsmoment wird dann laufend an das Modul 9 übermittelt, welches dann das tatsächlich auf das Fluid wirkende Drehmoment ermitteln kann.

Das so korrigierte Drehmoment wird dann an die Module 11 und 12 zur Ermittlung einer korrigierten Durchflussrate und eines korrigierten Differenzdrucks übermittelt.

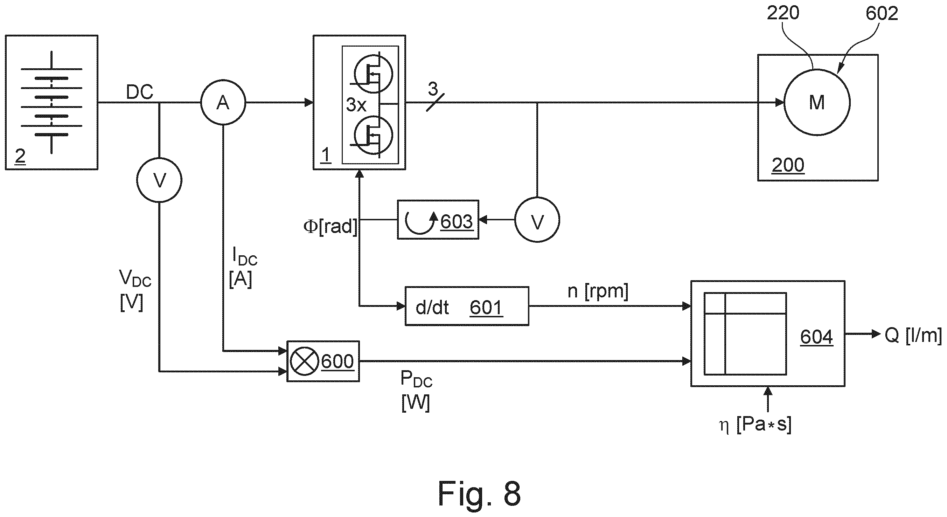

Figur 8 zeigt die Funktionsweise einer Flussschätzung, wie sie zum Zeitpunkt der Anmeldung bei implantierten mechanischen Herzunterstützungssystemen marktüblich ist. Die Pumpe 200 wird vom Motor 220 angetrieben, welcher vom Motortreiber 1 angesteuert wird. Der Motortreiber benötigt hierfür eine Information über die Rotorposition, welche diesem über eine Phasenspannungsmessung und einen Gegeninduktionsspannungs-Nulldurchgangsdetektor 603 bereitgestellt wird. Der Detektor 603 kann pro Umdrehung und Phase jeweils zwei Nulldurchgänge detektieren, also insgesamt 6 Detektionen pro Umdrehung des Motors. Den Detektionen kann ein sprungweise fortlaufender Rotorwinkel Φ zugeordnet werden. Zu jedem Rotorwinkelsprung schaltet der Motortreiber 1 im Betriebsmodus der Blockkommutierung die drei Motorphasen in den nächsten Kommutierungszustand. Für die Flussschätzung werden Motortreiber 1 und Motor 220 als Einheit betrachtet. An der Verbindungsstelle dieser Einheit zur Stromversorgung 2 wird nach einer Spannungs- sowie Strommessung in der Ermittlungseinheit 600 die Aufnahmeleistung bestimmt.

Die einfache Flussschätzungseinheit 604 ordnet einer Drehzahl n und einer Aufnahmeleistung $P_{DC}$ einen geschätzten Fluss Q zu. Der reale Fluss hängt von weiteren Parametern ab, welche in der einfachen Flussschätzung als konstant angenommen werden. Einen großen Einfluss hat die Viskosität η des Förderfluids, welche üblicherweise vom Pumpen-Operator manuell eingestellt wird. Weitere Parameter wie der Wirkungsgrad des Motors sind in der Flussschätzeinheit 604 fest eingespeichert. Auf den Motor 220 oder den Motortreiber 1 wirkende Störeinflüsse 602, die den Wirkungsgrad beeinflussen, reduzieren die Genauigkeit der Flussschätzeinheit 604. Die Störeinflüsse 602 können beispielsweise eine Veränderung der Spulentemperatur, Magnettemperatur oder der Position der Rotormagnete sein. Der Wirkungsgrad des Motortreibers kann ebenfalls durch Temperatur oder Alterung variieren.

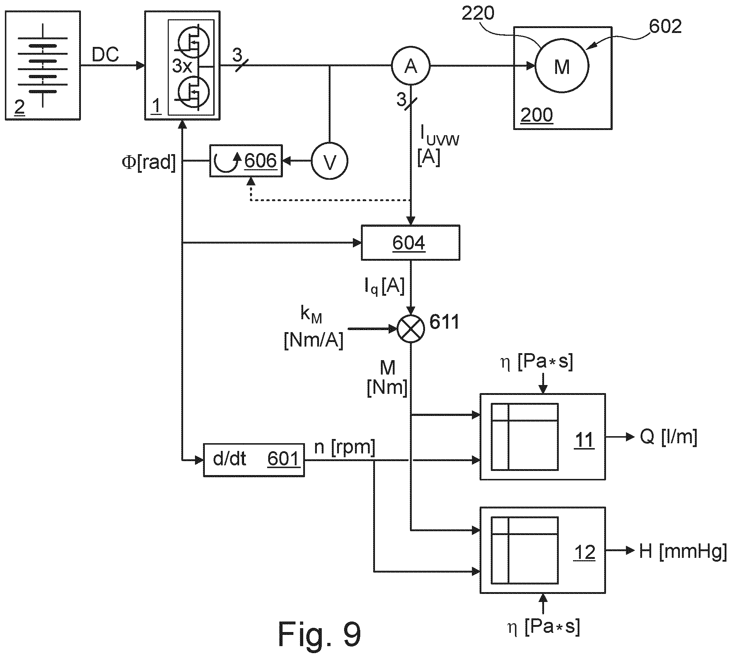

Figur 9 zeigt eine Verbesserung gegenüber der Ausführungsform der Figur 8. Die Genauigkeit der Flussschätzeinheit 11 hängt hierbei nicht mehr vom Wirkungsgrad des Motortreibers 1 ab. Dies wird dadurch erreicht, dass Phasenströme des Motors gemessen werden und mit einer Clarke-Park-Transformation 604 der tatsächlich in ein Drehmoment umgesetzte Stromanteil $I_q$ (Drehmomentstrom) bestimmt wird. Damit die Bestimmung des Drehmomentstroms $I_q$ genau wird, ist es vorteilhaft, den Motortreiber und den Motor in Sinuskommutierung oder mit einer feldorientierten Regelung zu betreiben. Dabei wird der Motor nicht blockweise sondern kontinuierlich angetrieben. Dadurch kann der Gegeninduktionsspannungsdetektor 606 einen genaueren und kontinuierlichen Rotorwinkel Φ bestimmen, insbesondere, wenn neben den Phasenspannungen auch die Phasenströme in dem Gegeninduktionsspannungsdetektor 606 berücksichtigt werden.

Aus dem gemessenen Drehmomentstrom $I_q$ wird das Motorabgangsmoment M im Schritt 611 durch Multiplikation mit der Drehmomentkonstante $k_M$ bestimmt. Die Drehmomentkonstante $k_M$ ist üblicherweise fest eingestellt. Vorteilhaft gegenüber einem fest eingestellten Wirkungsgrad wie gemäß Figur 8, werden in der Ausführungsform gemäß Figur 9 neben dem Wirkungsgrad des Motortreibers auch einige Störungen 602 des Motors, wie beispielsweise die Änderungen der Spulentemperatur, ausgeglichen. Andere Störungen, wie beispielsweise Abweichungen der Rotorposition oder Rotormagnetposition von einer Idealposition, verringern die Genauigkeit der Flussschätzung weiterhin. Aus denselben Eingangswerten der Flussschätzeinheit 11 kann auch eine Druckschätzeinheit 12 mit den notwendigen Informationen versorgt werden.

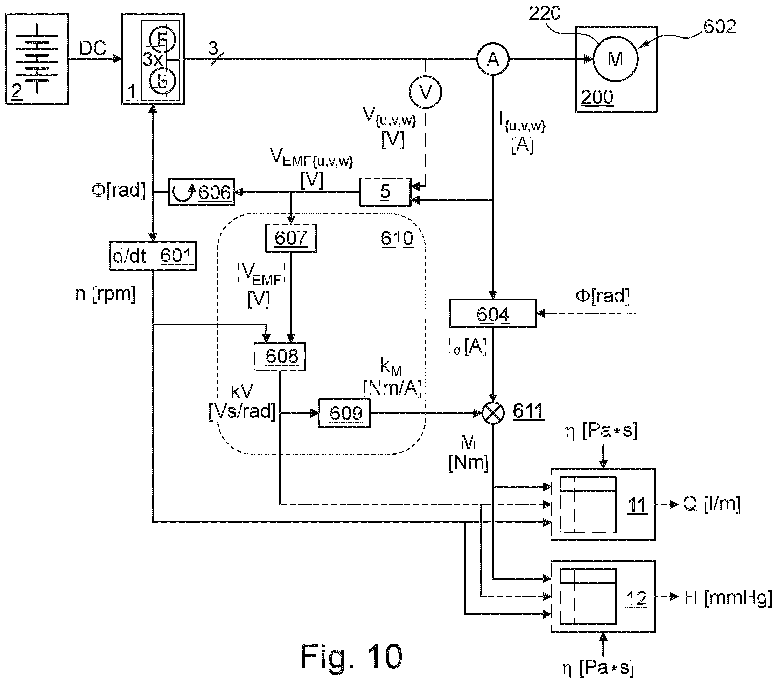

Figur 10 zeigt eine weitere Verbesserung gegenüber der in Figur 9 gezeigten Ausführungsform, mit der alle Störungen 602 kompensiert werden können und somit das Motorabgangsmoment des Motors 220 genauer bestimmt werden kann. Hierfür wird ausgenutzt, dass die Drehmomentkonstante $k_M$ im laufenden Betrieb aus der Drehzahl und der Gegeninduktionsspannung bestimmt werden kann. Dies erfordert eine besonders genaue Messung der Gegeninduktionsspannung, insbesondere auch bei veränderlichen Phasenwiderständen oder Phaseninduktivitäten. Die vorab beschriebene und in den Figuren 3, 4 und 5 dargestellte Methode der Bestimmung der Gegeninduktionsspannung durch Nachführung der Phasenimpedanz ist im Block 5 realisiert. Aus den Gegeninduktionsspannungen aller Phasen lässt sich, beispielsweise durch Vektoraddition und Betragsbildung, im EMF-Betragsermittler 607 eine Amplitude $|V_{EMF}|$ bestimmen. Die Bestimmung der Drehzahl n kann durch die zeitliche Ableitung des gemessenen Phasenwinkels Φ in dem Schritt 601 erfolgen. Aus dem Verhältnis von n und $|V_{EMF}|$ kann im Schritt 608 die Gegeninduktionskonstante $k_V$ bestimmt werden. Zwischen der Gegeninduktionskonstante $k_V$ und Drehmomentkonstante $k_M$ besteht ein eindeutiger Zusammenhang, welcher in 609 abgebildet wird. Sämtliche Effekte und Störungen 602 welche die Gegeninduktion des Motors 220 beeinflussen, wie Rotorposition oder Rotormagnetstärke, können somit kompensiert werden. Das von den Motorspulen ausgehende Abtriebsmoment kann damit genau bestimmt werden. Die Schätzeinheiten 11 und 12 können nun von einem genau ermittelten Motorabtriebsmoment ausgehen und erhöhen somit ihre Genauigkeit. In den Verarbeitungseinheiten/-Schätzern 11 und 12 sind die HQ-Kennlinien der Strömungsmaschine hinterlegt. Die Viskosität η beeinflusst die HQ-Kennlinien nicht nur bezüglich der Position, sondern in geringem Maß auch bezüglich der Form. Die genaue Drehmomentbestimmung ermöglicht es somit auch, die Viskosität η zu schätzen und nachzuführen, anstatt diese manuell eingeben zu müssen. Die gestrichelte Linie 610 fasst einige Komponenten zusammen, die die Anordnung gemäß Figur 10 von der in Figur 9 gezeigten Lösung unterscheiden.

Mit der beschriebenen Steuereinheit sowie dem Verfahren für eine Rotationsfluidpumpe wird eine genaue, zuverlässige und langzeitstabile Bestimmung des Durchflusses und/oder des Differenzdrucks möglich.

## Patentansprüche

1. Steuereinheit (300) für eine Rotationsfluidpumpe (200), insbesondere eine Blutpumpe, wobei die Rotationsfluidpumpe (200) einen zum Fördern von Fluid um eine Rotationsachse (500) rotierbaren Rotor (240) sowie einen Stator (220) mit einer Mehrzahl von Motorspulen (221), umfasst, wobei die Steuereinheit (300) eingerichtet ist zum:

   Erzeugen elektrischer Antriebssignale, mit denen die Motorspulen beaufschlagt werden,
   Erfassen und/oder Bestimmen von Phasenstromwerten und Phasenspannungswerten für eine oder mehrere Motorspulen,
   Ermittlung der Gegeninduktionsspannung aus den Phasenstromwerten und den Phasenspannungswerten,
   Erfassen und/oder Bestimmen der Drehzahl des Rotors
   Ermittlung eines Gegeninduktionskoeffizienten kv aus der Gegeninduktionsspannung und der Drehzahl,
   Ermitteln des Drehmomentes des Rotors aus dem Gegeninduktionskoeffizienten und den Phasenstromwerten, sowie
   Ermittlung, insbesondere Schätzung, des Durchflusses und/oder des Differenzdrucks der Rotationsfluidpumpe unter Berücksichtigung der Drehzahl und des Drehmomentes des Rotors.

2. Steuereinheit nach Anspruch 1, **dadurch gekennzeichnet, dass** die Steuereinheit (300) eingerichtet ist, im Betrieb wiederholt, insbesondere regelmäßig, den Gegeninduktionskoeffizienten kv aus der Gegeninduktionsspannung und der Drehzahl zu ermitteln, und damit insbesondere Veränderungen der Motoreffizienz und/oder der Rotorposition und/oder der Rotortemperatur und/oder der Alterung der Rotormagnete und/oder produktionsbedingte Bauteilstreuungen mit Auswirkung auf die Motoreffizienz bei der Rotordrehmomentbestimmung zu berücksichtigen.

3. Steuereinheit nach Anspruch 1 oder 2, **dadurch gekennzeichnet, dass** die Steuereinheit (300) eingerichtet ist, aus dem Gegeninduktionskoeffizienten und den Phasenströmen ein Motorspulenangangsmoment zu bestimmen, und aus diesem durch Berücksichtigung von nicht hydrodynamischen Bremsmomenten, insbesondere Wirbelstromverlusten und/ oder Hystereseverlusten, das tatsächlich auf das zu fördernde Fluid wirkendende Drehmoment zu bestimmen.

4. Steuereinheit nach Anspruch 1, 2 oder 3, **dadurch gekennzeichnet, dass** die Steuereinheit (300) eingerichtet ist zum:

   Erzeugen eines Steuersignals für mindestens eine der Mehrzahl von Motorspulen (221), wobei das Steuersignal mit einem Modulationssignal beaufschlagt wird,
   Erfassen mindestens eines Messsignals, entsprechend einem durch die mindestens eine der Mehrzahl von Motorspulen (221) fließenden Strom und/oder einer an der mindestens einen der Mehrzahl von Motorspulen (221) anliegenden Spannung,
   Demodulieren des mindestens einen Messsignals zum Bestimmen mindestens eines veränderlichen charakteristischen elektrischen Widerstands einer elektrischen Anordnung, umfassend die mindestens eine der Mehrzahl von Motorspulen (221) und eine zum Verbinden der mindestens einen der Mehrzahl von Motorspulen (221) mit der Steuereinheit (300) eingerichtete Zuleitung und/oder
   wobei die Steuereinheit (300) eingerichtet ist zum:

   Erfassen mindestens eines Messsignals, entsprechend einem durch mindestens eine der Mehrzahl von Motorspulen (221) fließenden Strom und/oder einer an der mindestens einen der Mehrzahl von Motorspulen (221) anliegenden Spannung,
   Bestimmen, basierend auf dem mindestens einen Messsignal und einem thermischen Modell, einer Änderung einer Temperatur wenigstens eines Teils der Rotationsfluidpumpe (200), umfassend die mindestens eine der Mehrzahl von Motorspulen (221), über ein Messintervall; und
   Bestimmen, basierend auf der bestimmten Änderung der Temperatur, eines Wertes oder einer Änderung mindestens eines veränderlichen charakteristischen elektrischen Widerstands einer elektrischen Anordnung, umfassend die mindestens eine der Mehrzahl von Motorspulen (221) und eine zum Verbinden der mindestens einen der Mehrzahl von Motorspulen (221) mit der Steuereinheit (300) eingerichtete Zuleitung.

5. Steuereinheit (300) nach Anspruch 4, dazu eingerichtet,

den mindestens einen veränderlichen charakteristischen elektrischen Widerstand in einem Normalbetrieb der Rotationsfluidpumpe (200) wiederholt für jeweils ein erstes Messintervall durch Demodulieren des mindestens einen Messsignals zu bestimmen, und
eine Änderung des mindestens einen veränderlichen charakteristischen elektrischen Widerstands über ein zweites Messintervall, das kürzer ist als das erste Messintervall, basierend auf der bestimmten Änderung der Temperatur zu bestimmen, insbesondere in Antwort auf ein Erfassen eines gegenüber dem Normalbetrieb erhöhten Leistungseintrags in die Rotationsfluidpumpe (200).

6. Steuereinheit (300) nach einem der Ansprüche 1 bis 5, wobei der Stator (220) eine Mehrzahl von Motorphasen, umfassend jeweils mindestens eine der Mehrzahl von Motorspulen (221), umfasst und die Steuereinheit (300) dazu eingerichtet ist,

nacheinander eine Mehrzahl von Modulationszuständen vorzugeben, wobei in jedem Modulationszustand ein jeweiliger Satz von Motorphasen gegenphasig oder phasenverschoben mit dem Modulationssignal beaufschlagt wird und
wobei das Messsignal im jeweiligen Modulationszustand an dem jeweils mit dem Modulationssignal beaufschlagten Satz von Motorphasen abgegriffen wird.

7. Steuereinheit nach einem der Ansprüche 1 bis 6, **dadurch gekennzeichnet, dass** die Steuereinheit dazu eingerichtet ist, aus dem Gegeninduktionskoeffizienten eine Lagerposition des Rotors in seiner Axialrichtung zu ermitteln und diese insbesondere bei der Ermittlung oder Schätzung des Flusses und/oder des Differenzialdrucks der Rotationsfluidpumpe und/oder bei der Lagerregelung zu berücksichtigen.

8. Steuereinheit nach einem der Ansprüche 1 bis 7, **dadurch gekennzeichnet, dass** die Steuereinheit eine Zuordnungseinheit aufweist, die dazu eingerichtet ist, aufgrund eines Zuordnungsmodells einem ermittelten Drehmoment und einer Drehzahl des Rotors, insbesondere unter Berücksichtigung einer Lagerposition des Rotors und/oder eines Viskositätswertes des geförderten Fluids einen Durchfluss und/oder einen Differenzdruck der Rotationsfluidpumpe zuzuordnen, wobei das Zuordnungsmodell insbesondere eine Zuordnungstabelle, eine Zuordnungsfunktion, einen Beobachter oder ein selbstlernendes System, insbesondere ein neuronales Netz, umfasst.

9. Pumpensystem (100), umfassend
eine Rotationsfluidpumpe (200), umfassend einen zum Fördern von Fluid um eine Rotationsachse (500) rotierbaren Rotor (240) sowie einen Stator (220) mit einer Mehrzahl von Motorspulen (221), und eine Steuereinheit nach einem der Ansprüche 1 bis 8.

10. Verfahren zum Ermitteln eines Durchflusses und/oder eines Differenzdrucks einer Rotationsfluidpumpe (200), insbesondere einer Blutpumpe, wobei die Rotationsfluidpumpe (200) einen zum Fördern von Fluid um eine Rotationsachse (500) rotierbaren Rotor (240) sowie einen Stator (220) mit einer Mehrzahl von Motorspulen (221), umfasst, mittels einer Steuereinheit (300), wobei die Steuereinheit

elektrische Antriebssignale erzeugt, mit denen die Motorspulen beaufschlagt werden,
Phasenstromwerte und Phasenspannungswerte für eine oder mehrere Motorspulen erfasst oder bestimmt,
eine Gegeninduktionsspannung aus den Phasenstromwerten und den Phasenspannungswerten ermittelt,
die Drehzahl des Rotors erfasst oder bestimmt,
einen Gegeninduktionskoeffizienten kv aus der Gegeninduktionsspannung und der Drehzahl ermittelt,
das Drehmoment des Rotors aus dem Gegeninduktionskoeffizienten und den Phasenstromwerten ermittelt, sowie
den Durchfluss und/oder den Differenzdruck der Rotationsfluidpumpe unter Berücksichtigung der Drehzahl und des Drehmomentes des Rotors ermittelt, insbesondere schätzt.

11. Verfahren nach Anspruch 10, **dadurch gekennzeichnet, dass** die Steuereinheit (300) im Betrieb wiederholt, insbesondere regelmäßig, den Gegeninduktionskoeffizienten kv aus der Gegeninduktionsspannung und der Drehzahl ermittelt, und damit insbesondere Veränderungen der Motoreffizienz und/oder der Rotorposition und/oder der Rotortemperatur und/oder der Alterung der Rotormagnete bei der Rotordrehmomentbestimmung berücksichtigt.

12. Verfahren nach Anspruch 10 oder 11, **dadurch gekennzeichnet, dass** die Steuereinheit (300)

ein Steuersignal für mindestens eine der Mehrzahl von Motorspulen (221) erzeugt, wobei das Steuersignal mit einem Modulationssignal beaufschlagt wird, mindestens ein Messsignal erfasst, das einen durch die mindestens eine der Mehrzahl von Motorspulen (221) fließenden Strom und/oder eine an der mindestens einen der Mehrzahl von Motorspulen (221) anliegende Spannung repräsentiert, mindestens ein Messsignal zum Bestimmen mindestens eines veränderlichen charakteristischen elektrischen Widerstands einer elektrischen Anordnung demoduliert, wobei die Anordnung mindestens eine der Mehrzahl von Motorspulen (221) und eine zum Verbinden der mindestens einen der Mehrzahl von Motorspulen (221) mit der Steuereinheit (300) eingerichtete Zuleitung umfasst, und/oder , dass die Steuereinheit (300): mindestens ein Messsignal erfasst, das einen durch mindestens eine der Mehrzahl von Motorspulen (221) fließenden Strom und/oder eine an der mindestens einen der Mehrzahl von Motorspulen (221) anliegende Spannung repräsentiert, basierend auf dem mindestens einen Messsignal und einem thermischen Modell eine Änderung einer Temperatur wenigstens eines Teils der Rotationsfluidpumpe (200), über ein Messintervall bestimmt, wobei der mindestens eine Teil wenigstens eine der Mehrzahl von Motorspulen (221) umfasst; und basierend auf der bestimmten Änderung der Temperatur, einen Wert oder eine Änderung mindestens eines veränderlichen charakteristischen elektrischen Widerstands einer elektrischen Anordnung bestimmt, wobei die elektrische Anordnung die mindestens eine der Mehrzahl von Motorspulen (221) und eine zum Verbinden der mindestens einen der Mehrzahl von Motorspulen (221) mit der Steuereinheit (300) eingerichtete Zuleitung umfasst.

13. Verfahren nach Anspruch 12, **dadurch gekennzeichnet, dass** die Steuereinheit (300)

den mindestens einen veränderlichen charakteristischen elektrischen Widerstand in einem Normalbetrieb der Rotationsfluidpumpe (200) wiederholt für jeweils ein erstes Messintervall durch Demodulieren des mindestens einen Messsignals bestimmt, und eine Änderung des mindestens einen veränderlichen charakteristischen elektrischen Widerstands über ein zweites Messintervall, das kürzer ist als das erste Messintervall, basierend auf der bestimmten Änderung der Temperatur bestimmt, insbesondere nach dem Erfassen eines gegenüber dem Normalbetrieb erhöhten Leistungseintrags in die Rotationsfluidpumpe (200).

14. Verfahren nach einem der Ansprüche 10 bis 13, wobei der Stator (220) eine Mehrzahl von Motorphasen, umfassend jeweils mindestens eine der Mehrzahl von Motorspulen (221), umfasst und die Steuereinheit (300)

nacheinander eine Mehrzahl von Modulationszuständen vorgibt, wobei in jedem Modulationszustand ein jeweiliger Satz von Motorphasen gegenphasig oder phasenverschoben mit dem Modulationssignal beaufschlagt wird und wobei das Messsignal im jeweiligen Modulationszustand an dem jeweils mit dem Modulationssignal beaufschlagten Satz von Motorphasen abgegriffen wird.

15. Verfahren nach einem der Ansprüche 10 bis 14, **dadurch gekennzeichnet, dass** die Steuereinheit aus dem Gegeninduktionskoeffizienten eine Lagerposition des Rotors in seiner Axialrichtung ermittelt und diese insbesondere bei der Ermittlung oder Schätzung des Flusses und/oder des Differenzialdrucks der Rotationsfluidpumpe und/oder bei der Lageregelung berücksichtigt.

Fig. 1

Fig. 2

Fig. 3

Fig. 4

Fig. 5

Fig. 6

Fig. 7

Fig. 8

Fig. 9

Fig. 10

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 6 135 943 A (YU YIH-CHOUNG [US] ET AL) 24. Oktober 2000 (2000-10-24) | 1,8-10 | INV. F04D15/00 |
| A | * Spalte 2, Zeilen 46-61 * <br> * Spalte 3, Zeilen 52-55 * <br> * Spalte 4, Zeile 53 - Spalte 5, Zeile 20 * <br> * Spalte 6, Zeile 46 - Spalte 7, Zeile 10 * <br> ----- | 2-7, 11-15 | A61M60/422 A61M60/538 |
| A | DE 697 33 746 T2 (THORATEC CORP [US]) 8. Juni 2006 (2006-06-08) <br> * Absätze [0025] - [0029] * <br> ----- | 1 | |
| A | WO 2023/180325 A1 (BERLIN HEART GMBH [DE]) 28. September 2023 (2023-09-28) <br> * Anspruch 13 * <br> ----- | 7,15 | |

RECHERCHIERTE SACHGEBIETE (IPC)

F04D
A61M

Der vorliegende Recherchenbericht wurde für alle Patentansprüche erstellt

| Recherchenort | Abschlußdatum der Recherche | Prüfer |
|---|---|---|
| Den Haag | 29. Mai 2025 | Brouillet, Bernard |

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

**EP 24 22 1942**

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

**29-05-2025**

| Im Recherchenbericht angeführtes Patentdokument | Datum der Veröffentlichung | Mitglied(er) der Patentfamilie | Datum der Veröffentlichung |
|---|---|---|---|
| US 6135943 A | 24-10-2000 | AT E499122 T1 | 15-03-2011 |
| | | AU 760987 B2 | 29-05-2003 |
| | | CA 2338204 A1 | 17-02-2000 |
| | | EP 1105172 A1 | 13-06-2001 |
| | | JP 2003526389 A | 09-09-2003 |
| | | US 6135943 A | 24-10-2000 |
| | | US 6447441 B1 | 10-09-2002 |
| | | WO 0007643 A1 | 17-02-2000 |
| DE 69733746 T2 | 08-06-2006 | AT E299721 T1 | 15-08-2005 |
| | | AU 713592 B2 | 09-12-1999 |
| | | CA 2241888 A1 | 14-05-1998 |
| | | DE 69733746 T2 | 08-06-2006 |
| | | EP 0877633 A2 | 18-11-1998 |
| | | ES 2243987 T3 | 01-12-2005 |
| | | JP 2000504977 A | 25-04-2000 |
| | | US 5888242 A | 30-03-1999 |
| | | US 6066086 A | 23-05-2000 |
| | | WO 9819624 A2 | 14-05-1998 |
| WO 2023180325 A1 | 28-09-2023 | CN 118891081 A | 01-11-2024 |
| | | DE 112023001469 A5 | 02-01-2025 |
| | | EP 4249040 A1 | 27-09-2023 |
| | | WO 2023180325 A1 | 28-09-2023 |

EPO FORM P0461

Für nähere Einzelheiten zu diesem Anhang : siehe Amtsblatt des Europäischen Patentamts, Nr.12/82